# EUROPÄISCHE PATENTSCHRIFT

(11) **EP 1 723 233 B1**
(45) Veröffentlichungstag und Bekanntmachung des Hinweises auf die Patenterteilung: **18.04.2012**
(21) Anmeldenummer: 05715667.1
(22) Anmeldetag: 02.03.2005
(51) Int. Cl.: C12N 15/10

(54) **VERFAHREN ZUR ANREICHERUNG UND/ODER ABTRENNUNG VON PROKARYOTISCHER DNA MITTELS EINES PROTEINS, DAS NICHT-METHYLIERTE CPG-MOTIVE ENTHALTENDE DNA SPEZIFISCH BINDET**
METHOD FOR ENRICHING AND/OR SEPARATING PROKARYOTIC DNA USING A PROTEIN THAT SPECIFICALLY BONDS TO UNMETHYLATED DNA CONTAINING CPG-MOTIVES
PROCEDE POUR CONCENTRER ET/OU SEPARER DE L'ADN PROCARYOTE AU MOYEN D'UNE PROTEINE QUI SE LIE DE MANIERE SPECIFIQUE A DE L'ADN NON METHYLE CONTENANT DES MOTIFS CPG

(30) Priorität: 05.03.2004 DE 102004010928; 14.01.2005 DE 102005001889
(43) Veröffentlichungstag der Anmeldung: 22.11.2006
(73) Patentinhaber: SIRS-Lab GmbH, 07745 Jena (DE)
(72) Erfinder: SCHMIDT, Karl-Hermann, 07646 Stadtroda (DE); STRAUBE, Eberhard, 07745 Jena (DE); RUSSWURM, Stefan, 07743 Jena (DE); DEIGNER, Hans-Peter, 68623 Lampertheim (DE); SACHSE, Svea, 07747 Jena (DE); LEHMANN, Marc, 07743 Jena (DE)
(74) Vertreter: Winter, Brandl, Fürniss, Hübner, Röss, Kaiser, Polte - Partnerschaft
(86) Internationale Anmeldenummer: PCT/EP2005/002198
(87) Internationale Veröffentlichungsnummer: WO 2005/085440

(56) Entgegenhaltungen:
- EP-A- 1 400 589
- SACHSE S ET AL: "Using a DNA-binding protein to enrich prokaryotic DNA from a mixture of both eukaryotic and prokaryotic DNA" 56. DGHM-JAHRESTAGUNG, 26.-29.SEPTEMBER 2004, MÜNSTER, DE, [Online] 2004, XP002332325 Gefunden im Internet: URL:http://www.sirs-lab.de/content/de/pdf/ kompetenzen/sachse.pdf> [gefunden am 2005-06-16]
- CROSS S H ET AL: "Purification of CpG islands using a methylated DNA binding column" NATURE GENETICS, NEW YORK, NY, US, Bd. 6, Nr. 3, 1. März 1994 (1994-03-01), Seiten 236-244, XP000578157 ISSN: 1061-4036
- VOO K S ET AL: "Cloning of a mammalian transcriptional activator that binds unmethylated CpG motifs and shares a CXXC domain with DNA methyltransferase, human trithorax, and methyl-CpG binding domain protein 1" MOLECULAR AND CELLULAR BIOLOGY, AMERICAN SOCIETY FOR MICROBIOLOGY, WASHINGTON, US, Bd. 20, Nr. 6, März 2000 (2000-03), Seiten 2108-2121, XP002262527 ISSN: 0270-7306

## Beschreibung

Die Erfindung betrifft Verfahren zur Trennung und/oder Anreicherung prokaryonter DNA bzw. zur Abreicherung dieser DNA aus physiologischen Flüssigkeiten unter Verwendung eines Proteins gemäß SEQ ID NO:2 das nicht-methylierte Cytidin-Phosphat-Guanosin-Dinukleotide (CpG-Motive) einer DNA spezifisch bindet, sowie die Verwendung eines Kitszur Durchführung des Verfahrens.

Durch Bakterien verursachte Infektionen sind eine der häufigsten Ursachen für Entzündungskrankheiten. Zur Prognose des Krankheitsverlaufes sowie insbesondere zur rechtzeitigen Auswahl geeigneter therapeutischer Maßnahmen ist der frühzeitige Nachweis der bakteriellen Erreger von entscheidender Bedeutung.

Zum Nachweis bakterieller Erreger werden auch heute noch hauptsächlich verschiedene kulturabhängige Methoden angewendet. Aktuelle Studien verdeutlichen jedoch die mangelnde Eignung von kulturabhängigen Methoden zum Erregernachweis (Hellebrand W., König-Bruhns C., Hass W., Studie zu Blutkulturdiagnostik im Jahr 2002, Poster Jahrestagung der Deutschen Gesellschaft für Hygiene und Mikrobiologie, Göttingen 2004; Straube E (2003) Sepsis - microbiological diagnosis. Infection 31:284). Demnach konnten nur bei ca. 15-16% aller untersuchten Blutkulturen die Erreger ermittelt werden. Die Nachteile dieser Methoden führten dazu, daß gerade in der letzten Dekade parallel zur stürmischen technologischen Entwicklung der Molekularbiologie verstärkt nach Alternativen gesucht wurde. Erste Berichte zum Einsatz kulturunabhängiger Nachweisverfahren bakterieller Erreger, welche auf dein Prinzip der Polymerasekettenreaktion (PCR) beruhen, stammen vom Anfang der 90er Jahre. So konnten beispielsweise Miller und Kollegen (Miller N J Clin Microbiol. 1994 Feb;32(2):393-7) zeigen, dass kulturunabhängige Verfahren den klassischen Kultivierungs- und Mikroskopietechniken beim Nachweis von *Mycobacterium tuberculosis* überlegen sind. In letzter Zeit haben aber weitere molekularbiologische Methoden; die auf dem Nachweis erregerspezifischer Nukleinsäuren basieren, an Bedeutung gewonnen (z.B. M. Gnjalva et al. Heart 89 (2003) 263-268; Uyttendaele M et al. Lett Appl Microbiol. 2003;37(5):386-91; Saukkoriipi A et al. Mol Diagn. 2003 Mar;7(1):9-15; Tzanakaki G et al. FEMS Immunol Med Microbiol. 2003 Oct 24;39(1):31-6;).

Neben der hohen Spezifität solcher molekularbiologischer Methoden ist der geringe Zeitbedarf als wesentlicher Vorteil gegenüber konventionellen kulturabhängigen Methoden zu nennen. Allerdings ist die Sensitivität des Nachweises prokaryonter DNA direkt aus Körperflüssigkeiten und nicht vorbehandeltem Untersuchungsmaterial im Vergleich zur Kultur der Mikroorganismen bislang viel zu gering. Eine für den direkten Erregernachweis aus nicht vorbehandeltem Untersuchungsmaterial ausreichende Menge an Nukleinsäuren von Bakterien wird auch im Bereich der 16S-rRNA- Analyse, mittels PCR der 16S Region auf dem bakteriellen Chromosom und der anschließenden Sequenzanalyse des PCR Fragmentes, nur eingeschränkt erreicht, da sich meist mehrere Kopien für den die 16S-rRNA kodierenden Abschnitt auf dem Chromosom befinden. Der direkte spezifische Erregernachweis mittels 16S-rRNA Analyse setzt voraus, dass sich nur eine Erreger-Spezies in der zu untersuchenden Probe befindet. Befinden sich verschiedene Erreger-Spezies in der Probe, ist ein spezifischer Nachweis über Sequenzzierung der 16S-rRNA Region nicht möglich, da die verwendeten Primer universell für die meisten Bakterien sind. Weiterhin ist für den Erregernachweis mittels 16S-rRNA-Analyse Voraussetzung, dass sich die nachzuweisenden Bakterien in der metabolischen Phase befinden und genügend 16S-rRNA exprimieren. Davon ist insbesondere bei Patienten, die unter einer kalkulierten antibiotischen Therapie stehen, in der Regel nicht auszugehen. Darüber hinaus kommen bestimmte Pathogenitätsfaktoren von Bakterien nicht zu jeder Zeit zur Expression, obwohl die entsprechenden Gene im bakteriellen Genom vorhanden sind. Im Ergebnis werden dem klinisch tätigen Arzt falsch negative Befunde übermittelt. Dadurch kann eine gezielte antibiotische Therapie entweder gar nicht oder viel zu spät eingeleitet werden. Der Arzt ist in solchen Fällen auf sein Erfahrungswissen und allgemeine Richtlinien (wie z.B. der Paul-Ehrlich-Gesellschaft) angewiesen und wird daher viel zu allgemein antibiotisch behandeln. Der nicht zielgenaue Einsatz von Antibiotika birgt eine Reihe von Risiken nicht nur für den einzelnen Patienten (wie z.B. unnötige Nebenwirkungen in Form von Nierenschäden etc.), sondern auch für die gesamte Gesellschaft (z.B. die Entwicklung zusätzlicher Antibiotikaresistenzen wie MRSA (Methicillinresistente *Staphylokokkus aureus,* etc.). Deshalb bietet der Nachweis der klinisch bedeutsamen Pathogenitätsfaktoren und Resistenzen von Bakterien auf chromosomaler und Plasmid-Ebene, also letztendlich auf DNA-Ebene, für die Diagnose vieler Infektionserkrankungen, aber auch der Sepsis, erhebliche Vorteile. Dies gilt um so mehr, da auf dieser Ebene auch eine Unterscheidung zwischen pathogenen und kommensalen Bakterien getroffen werden kann.

Am häufigsten erfolgt der erregerspezifische Nukleinsäurenachweis durch Nukleinsäure-Amplifikationstechniken (NAT), wie beispielsweise die Vervielfältigung der prokaryonten DNA mittels der Polymerase-Kettenreaktion (PCR) bzw. der Ligase-Kettenreaktion (LCR). Der hohen Spezifität und schnellen Verfügbarkeit der Ergebnisse stehen die Störanfälligkeit durch Kontaminationen oder stark reaktionshemmende Faktoren in klinischen Proben gegenüber.

Bei einem herkömmlichen PCR-Nachweisverfahren muß für eine erfolgreiche Detektion von Erregern im Blut theoretisch mindestens 1 Target-DNA des Erregers in 10 µl Blut vorhanden sein. Das entspricht ca. 100 Targets in 1 ml Blut bzw. 1000 Targets in 10 ml Blut. Anders verhält es sich bei der Blutkultur zum Nachweis von Erregern einer Infektion. Hier liegt die untere Nachweisgrenze bei etwa 3-5 Bakterien pro 10 ml Blut.

Diese Nachweisgrenze wird derzeit mit PCR-Verfahren noch nicht erreicht, auch nicht mit solchen, die ihre Zielsequenz im Bereich der 16S-rRNA Region auf dem Chromosom haben. Obwohl mehrere die 16S rRNA kodierende Regionen auf dem bakteriellen Chromosom lokalisiert sind, meist 3 bis 6, bleibt die Voraussetzung, daß sich mindestens ein Molekül der Template-DNA im PCR-Reaktionsgemisch befindet, unerfüllt.

Eine verbesserte diagnostische Sicherheit ist von PCR-Verfahren zu erwarten, deren spezifische Zielsequenzen für speziesspezifische Proteine kodieren, entweder im Chromosom oder auf Plasmiden der Mikroorganismen. Auch hier trifft das Obengesagte zur Nachweisgrenze zu. Gerade unter dem Einfluß einer laufenden Antibiotikatherapie kann das Wachstum der Erreger stark verlangsamt, eingeschränkt oder blockiert sein, auch wenn das eingesetzte Antibiotikum letztlich nicht optimal wirksam ist. Diese Situation ist gerade bei solchen Patienten häufig anzutreffen, die bereits unter Antibiotikatherapie stehen und bei denen aus diesem Grund keine krankheitsverursachenden Bakterien aus den Blutkulturen oder anderen Proben (wie z.B. Trachealabstrichen, bronchoalveolären Lavagen (BAL) etc.) angezüchtet werden können.

Wegen unzureichender Sensitivität hat der erregerspezifische Nukleinsäurennachweis ohne Amplifikationsschritt durch direkten Nachweis der prokaryonten DNA (Sondentechnik, FISH-Technik) nur bei ausreichend hoher Keimzahl im Untersuchungsmaterial diagnostische Bedeutung.

Die wesentliche Problematik des Nachweises prokaryonter DNA zur Identifikation bakterieller Erreger in Körperflüssigkeiten bestehen neben PCR-hemmenden Bestandteilen im Untersuchungsmaterial vor allem in der geringen Konzentration prokaryonter DNA und den damit einhergehenden Überschuß eukaryonter gegenüber prokaryonter DNA. Hierbei sind insbesondere kompetitive Prozesse bei der DNA-Analyse sowie die geringe Menge an prokaryonter DNA als hinderlich für einen qualitativen und quantitativen Erregernachweis anzusehen.

Die üblichen Methoden zur DNA-Isolierung reichern die Gesamt-DNA einer Körperflüssigkeit an, so daß das Verhältnis Wirts-DNA zu mikrobieller DNA zwischen 1:10⁻⁶ und 1:10⁻⁶ betragen kann. Aus diesem Unterschied ist die Schwierigkeit des Nachweises mikrobieller DNA in Körperflüssigkeiten gut nachzuvollziehen.

Prokaryonte DNA unterscheidet sich von eukaryonter DNA beispielsweise durch das Vorkommen nicht-methylierter CpG-Motive (Hartmann G et al., Deutsches Ärzteblatt, Jg. 98/15:A981-A985 (2001). In der prokaryonten DNA befinden sich CpG-Motive in einem 16-fachen Überschuß im Vergleich zu eukaryonter DNA, die solche Motive nur übergangsweise enthält, z.B. in Krebszellen oder Promotorregionen. In prokaryonter DNA sind diese Motive nicht methyliert, wohingegen sie in eukaryonter DNA zum größten Teil methyliert sind, was die Unterschiedlichkeit nochmals erhöht. Nicht-methylierte CpG-Motive sind nicht-methylierte Deoxycytidylat-Deoxyguanylat-Dinucleotide innerhalb des prokaryonten Genoms oder innerhalb von Fragmenten desselben.

Es ist weiterhin bekannt, dass aus unterschiedlichen Methylierungsmustern innerhalb der humanen DNA diagnostische Aussagen für Krebserkrankungen ableiten lassen (Epigenetics in Cancer Prevention: Early Detection and Risk Assessment (Annals of the New York Academy of Sciences, Vol 983) Editor: Mukesh Verma ISBN 1-57331-431-5). Anhand von methylierten und nicht-methylierten Cytosinen im Genom lassen sich gewebe-, aber auch krankheitsspezifische Muster identifizieren. Die spezifischen Methylierungsmuster für eine Krankheit ermöglichen zum einen eine Diagnose zu einem sehr frühen Zeitpunkt, zum anderen auch molekulare Klassifikation einer Krankheit und die wahrscheinliche Reaktion eines Patienten auf eine bestimmte Behandlung. Ausführliche informationen hierzu können beispielsweise aus Beck S, Olek A, Walter J.: From genomics to epigenomics: a loftier view of life.", Nature Biotechnology 1999 Dec;17(12):1144, der Homepage der Epigenomics AG (http://www.epigenomics.de) oder aus WO 200467775 entnommen werden.

In Cross et al. wurde gezeigt, dass es möglich ist, unterschiedlich methylierte genomische humane DNA zu trennen, indem die methylierte CpG-Motive an ein Protein gebunden werden (Cross SH, Charlton JA, Nan X, Bird AP, Purification of CpG islands using a methylated DNA binding column, Nat Genet. 1994 Mar;6(3):236-44). Dieses Verfahren dient also zur Bindung methylierte CpG-Motive enthaltende DNA. Eine ausreichende Trennung von nicht-methylierter und methylierter DNA ist aus technischen Gründen nicht möglich, da dass verwendete Protein auch nicht-methylierte DNA schwach bindet. Auch eine Anreicherung von nicht-methylierter DNA ist mit diesem Verfahren nicht möglich, da die Kapazität des verwendeten Proteins nicht ausreicht, um bei einem hohen Überschuss an methylierter DNA in ausreichendem Maße von nicht-methylierter DNA zu trennen. Weiterhin bleibt durch die Bindung der methylierten DNA das Ausgangsvolumen in welchem sich die nicht-methylierte DNA befindet unverändert, so dass keine Anreicherung erreicht wird.

Es wäre also wünschenswert, die nicht-methylierte DNA von methylierter DNA zu trennen und nicht-methylierte DNA anreichern zu können, um somit prokaryonte von eukaryonter DNA bzw. unterschiedlich methylierte humane DNA voneinander zu trennen. Es wäre zudem wünschenswert und von hohen gesundheitsökonomischen Interesse, dass die Trennung und Anreicherung von nicht-methylierter DNA auch aus einem Gemisch (beispielsweise Vollblut) erreicht werden könnte, dass durch einen hohen Überschuß an methylierter DNA charakterisiert ist.

Aus Voo et al. ist bekannt, dass das humane CpG-bindende Protein (hCGBP) in der Lage ist, nichtmethylierte CpG-Motive zu binden. Die Publikation beschreibt den transkriptionsaktivierenden Faktor hCGBP, von dem gezeigt wurde, dass er eine Rolle in der Regulation der Expression von Genen innerhalb von CpG-Motiven spielt.

In EP 02020904 wurde ein Verfahren gezeigt, das die Trennung und Anreicherung von prokaryonter DNA aus einem Gemisch aus prokaryonter und eukaryonter DNA durch Bindung der prokaryonten DNA an ein spezifisch nicht-methylierte DNA bindendes Protein ermöglicht.

Der vorliegenden Erfindung liegt somit die Aufgabe zugrunde, ein Verfahren bereitzustellen, die die Trennung und/oder Anreicherung prokaryonter DNA aus Untersuchungsproben mit hohem Anteil eukaryonter DNA, insbesondere von Patienten mit Infektionen, ermöglicht.

Erfindungsgemäß wird dies durch Verwendung eines Proteins gemäß SEQ ID NO:2 erreicht, das nicht methylierte CpG-Motive-bindet.

Als Wildtyp-CPGB-Protein (oder CGBP656) wird im folgenden das humane CGB-Protein (vgl. Voo et al., Mol Cell Biol. 2000 Mar; 20(6): 2108-21.) bezeichnet. Das im erfindungsgemäßen Verfahren verwendese Protein wird im folgenden als CGBP181 bezeichnet. Das in EP 02020904 beschriebene Protein, das eine verkürzte Variante des Wildtyp-CGB Proteins ist und als Grundlage für das Protein diente, wird im folgenden mit CGBP241 bezeichnet.

Die Erfindung wird nachfolgend unter Bezugnahme auf die Figuren beschrieben, wobei
- Figur 1: die Aminosäuresequenz von CGBP181 (fett dargestellt) verglichen mit dem Wildtyp-CPGB-Protein (CGBP656) und dem CGBP241 (kursiv dargestellt) zeigt;
- Figur 2: die DNA Sequenz und Übersetzung in die Aminosäuresequenz des kompletten CPG-bindenden Proteins CGBP656 zeigt, wobei die verkürzten CPG-bindenden Peptide CGBP241 (fett) und CGBP181 (kursiv) dargestellt sind;
- Figur 3: eine PCR von Streptokokken-DNA in Humanblut darstellt;
- Figur 4: eine nested PCR mit den PCR-Produkten aus dem Primär-PCR-Ansatz von Figur 3 als Template zeigt;
- Figur 5: ein Gelretadierungsexperiment darstellt
- Figur 6: ein weiteres Gelretardierungsexperiment wiedergibt;
- Figur 7: die Elution von Kalbsthymus-DNA und pUC18emm an hCGBP181-Sepharose zeigt, und
- Figur 8: die Darstellung der Bestimmung der eluierten DNA in den Fraktionen durch Messung der Extinktion bei 254 nm in Abhängigkeit des NaCl-Gradienten ist.
- Figur 9: Ergebnisse der PCR nach Anreicherung prokaryontischer DNA aus DNAGemisch von *Staphylococcus aureus* und human DNA unter Verwendung von gekoppeltem CGBP181 Protein an CNBr-Sepharose und
- Figur 10: Ergebnisse der PCR nach Anreicherung prokaryontischer DNA aus einem DNA-Gemisch von *Staphylococcus aureus* und humaner DNA unter Verwendung von gekoppeltem CGBP181-Protein an AH-Sepharose darstellen.

Das Wildtyp-CGB-Protein CGBP656 bindet nicht methylierte CpG-Motive prokaryonter DNA, wobei ein Protein-DNA-Komplex gebildet wird. Dieser kann z.B. auf einem Träger gebunden sein oder werden, wodurch eine Trennung und/oder Anreicherung der DNA erfolgen kann. Die vorliegende Erfindung basiert nun auf der überraschenden Erkenntnis, das ein im Vergleich zum Wildtyp CGB-Protein (CGBP656 mit 656 Aminosäuren) verkürztes Protein gemäß SEQ ID NO:2, eine verbesserte Bindungseigenschaft gegenüber nicht methylierten CpG-Motive prokaryonter DNA als das Wildtyp CGBP-Protein und Varianten davon mit 80% oder mehr Homologie besitzt. Beispiel eines solchen verkürzten Proteins ist CGBP181 mit 181 Aminosäuren.

Prokaryonte DNA unterscheidet sich von eukaryonter DNA beispielsweise durch das Vorkommen nicht methylierter CpG-Motive (Deutsches Ärzteblatt, Jg. 98/15: A981-A985 (2001)). Die Erfindung basiert auf der Kenntnis, daß sich eukaryonte DNA und prokaryonte DNA durch ihren Anteil an CpG-Motiven unterscheiden. In der prokaryonten DNA befinden sich CpG-Motive in einem 20-fachen Überschuß im Vergleich zu eukaryonter DNA, die solche Motive nur übergangsweise enthält, z.B. in Krebszellen oder Promotorregionen ((Deutsches Ärdeblatt, Jg. 98/15: A981-A985 (2001)). In prokaryonter DNA sind diese Motive nicht methyliert, wohingegen sie in eukaryonter. DNA zum größten Teil methyliert sind, was die Unterschiedlichkeit nochmals erhöht. Nicht methylierte CpG-Motive sind nicht methylierte Deoxycytidylat-Deoxyguanylat-Dinucleotide innerhalb des prokaryonten Genoms oder innerhalb von Fragmenten desselben.

Des weiteren basiert die Erfindung auf der Kenntnis, daß das gemäß SEQ ID No.2 Protein spezifisch an nicht methylierte CpG-Motive bindet. Diese spezifische Bindungseigenschaft des Proteins wird genutzt, um prokaryonte DNA zu binden und dadurch nachfolgend aus einer Probe z.B. mit überwiegendem Anteil eukaryonter DNA anzureichern, zu trennen und zu isolieren.

Die Bezeichnung "nicht-methylierte CpG-Motive enthaltende DNA bezieht sich dabei sowohl auf eukaryonte als auch prokaryonte DNA. Diese kann aufgereinigt und wieder in Lösung gebracht sein (z.B. aus Geweben isolierte nicht-methylierte DNA) oder direkt in der Ursprungsquelle (z. B. Körperflüssigkeit, wie Blut, Serum, Trachealaspirat, Urin, bronchalveoläre Lavage, Nasenabstrich, Hautabstrich, Punktionsflüssigkeit) vorliegen.

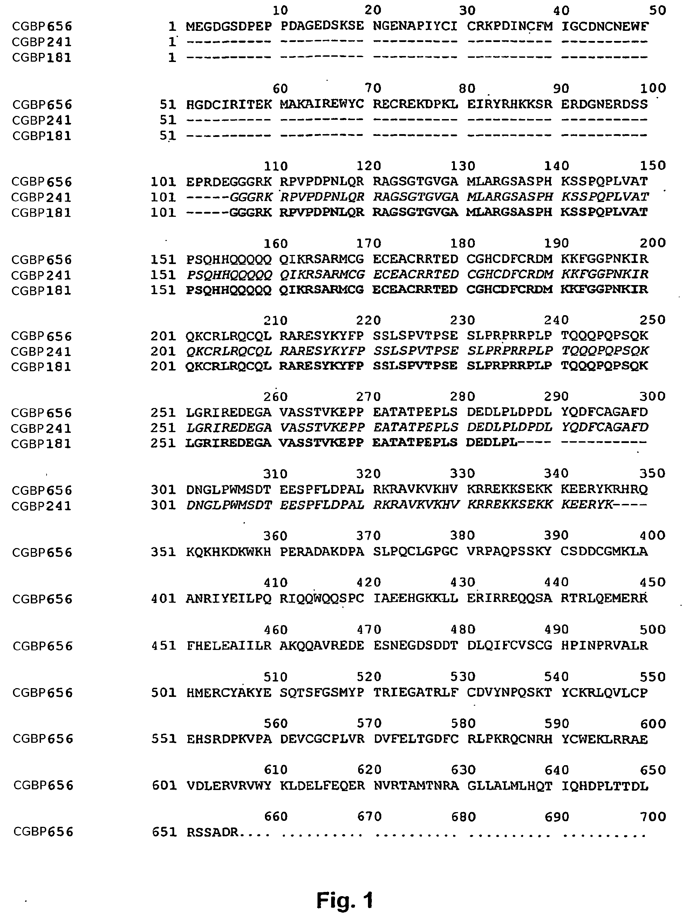

Gemäß einer bevorzugten Ausführungsform ist die nicht-methylierte CpG-Motive enthaltende DNA prokaryonte DNA, insbesondere bakterielle DNA. wie er zur Charakterisierung des Proteins gemäß SEQ ID No.2 verwendet wird, bedeutet, daß die Länge der Aminosäuresequenz des Proteins (CGBP181) kürzer als die Länge der Aminosäuresequenz des Wildtyp-CGB-Proteins () ist. Die Verkürzung erfolgt am N-terminalen und am C-terminalen Ende der Wildtyp-Proteinsequenz (Figur 1). Die maximale Verkürzung stellt dabei die DNA-Bindestelle des Proteins dar.

Das eingesetzte Protein kann z. B. ein Molekulargewicht von etwa 19959 Dalton (nativ) bzw. 21444 Dalton (im Plasmid pQE60) aufweisen. Der isoelektrische Punkt des Proteins kann etwa 10,09 (natives Protein) bzw. 10,15 (im Plasmid pQE60) betragen. Das im erfindungsgemäßen Verfahren eingesetzte Protein besitzt die in SEQ ID No. 2 bzw. Fig. 1 dargestellte Aminosäuresequenz. Dieses hat besonders gute Bindungseigenschaften gegenüber nicht methylierten CpG-Motiven prokaryonter DNA.

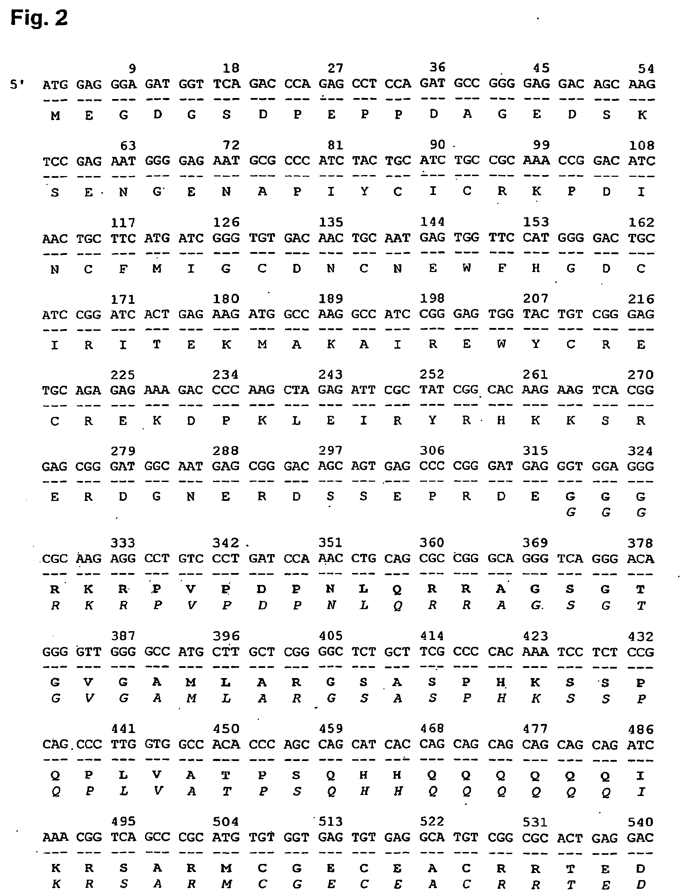

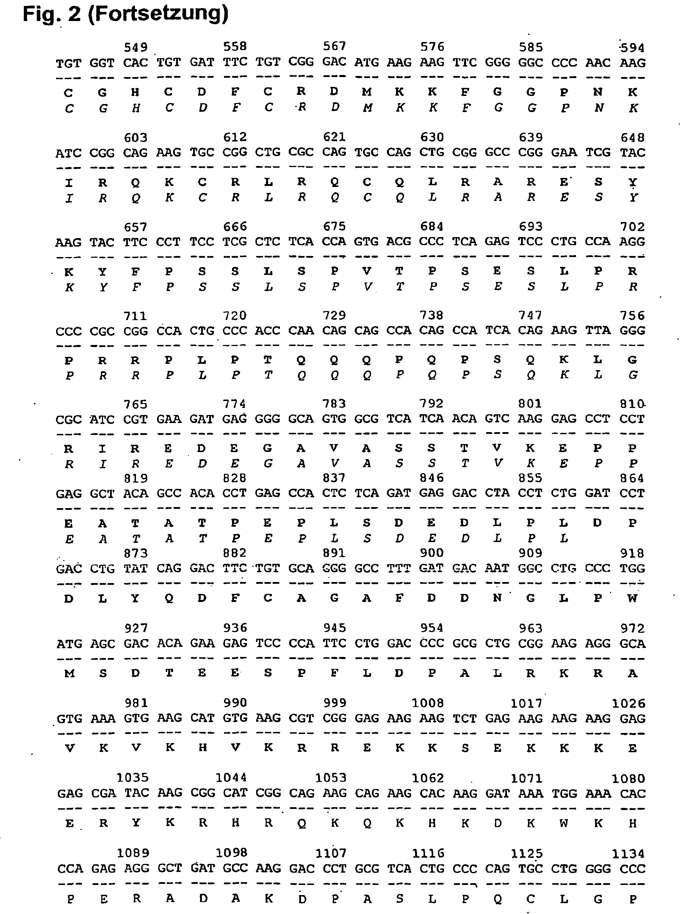

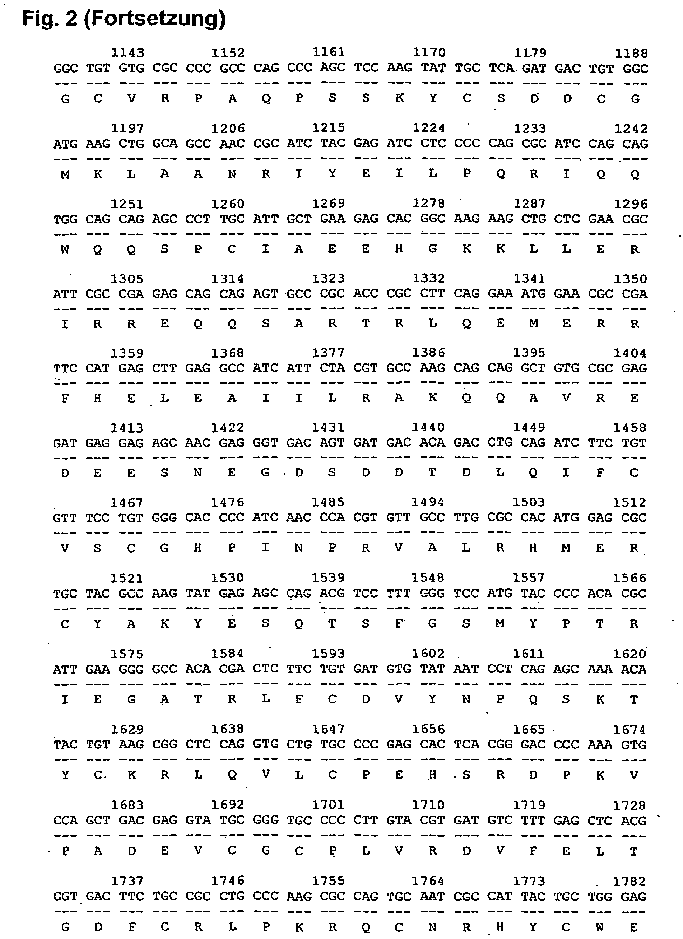

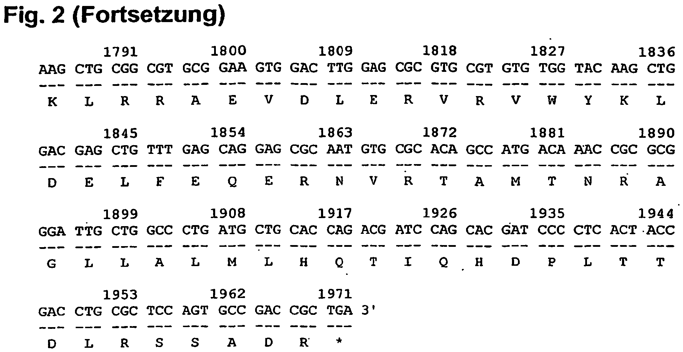

Das in EP 02020904 beschriebene Protein (CGBP241), das eine verkürzte Variante des Wildtyp-CPGB-Proteins (CGBP656) ist und als Grundlage für das Protein (CGBP181) diente, hat eine Länge von 241 Aminosäuren, ein Molekulargewicht von etwa 33650. Dalton (nativ) bzw. 28138 Dalton (im Plasmid pQE60) und einen isoelektrischen Punkt von 9,89 (nativ) bzw. 9,88 (im Plasmid pQE60). Die cDNA- und Aminosäuresequenz ist in Fig. 1 und 2 gezeigt.

Das Wildtyp-CGB-Protein hat eine Länge von 656 Aminosäuren, 135 positiv und 94 negativ geladene Reste, ein Molekulargewicht von etwa 75684 Dalton und einen isoelektrischen Punkt von 8,15. Die cDNA- und Aminosäuresequenz ist in Fig.1 gezeigt.

Der Sequenzvergleich des Proteins CGBP181 gemäß SEQ ID No. 2 mit dem in EP 02020904 beschriebenen Protein (CGBP241) ist in Fig. 1 und 2 dargestellt.

Das Protein wird gemäß SEQ ID No.2 wird bevorzugt durch Klonierung der entsprechenden cDNA-Sequenz in ein Plasmid und Expression in *Escherichia coli* hergestellt. Ein das Protein exprimierender *E.coli*-Stamm wurde am 16. Februar 2004 unter der Nr. DSM 16229 bei der Deutschen Sammlung für Mikroorganismen und Zellkulturen hinterlegt. Alternativ können andere, dem Fachmann vertraute Verfahren zur Herstellung angewendet werden. Die Nutzung des Plasmids pQE9 stellt dabei eine beispielhafte Möglichkeit dar, aber jedes andere geeignete Plasmid ist als Vektor einsetzbar. Die Expression in *E. coli* stellt ebenfalls nur ein Beispiel dar. Eine Expression ion anderen prokaryonten System als auch in einem eukaryonten System als auch die chemische oder enzymatische Synthese oder die Aufreinigung aus einer natürlichen Quelle, wie zB. Tabakpflanzen, sind weitere möglich Ausführungsformen der Proteingewinnung. Das Protein kann sowohl im Labormaßstab (z.B. im Erlenmeyerkolben) als auch im industriellen Maßstab (z.B. Fermenter) hergestellt werden. Das erfindungsgemäße Protein kann z.B. mittels Bindung von an den Anfang oder das Ende des Proteins eingebrachten Histidin-Reste (His-tag) an eine geeignete nickelhaltige Matrix gereinigt werden, eine Methode, die dem Fachmann bekannt ist.

Weitere Möglichkeiten der Reinigung können jegliche Art von Fusionsproteinen sein, die eine Aufreinigung über geeignete Matrices (Säulen, Gele, Beads etc.) erlauben. Andere Formen von tag's können Fusionspeptide / -proteine. z.B. Streptavidin-tag. Myc-tag und andere sein.

Eine bevorzugte Form des Proteins gemäß Seq. ID No.2 ist die native Form, aber auch eine denaturierte Form ist zur Bindung nicht methylierter CpG-Motive geeignet. Unter "denaturierten Formen" im Sinne der vorliegenden Erfindung werden andere Sekundärstrukturen als die in der Natur vorkommenden verstanden.

Die native oder auch denaturierte Form des Proteins stellt eine beispielhafte Ausführungsform dar. Die Erfindung schließt die *in vitro*-Synthese sowie alle weiteren chemischen oder enzymatigchen Modifikation des Proteins ein, wie z.B. Einbau von Disulfidbrücken, Glykosylierungen, Phosphorylierungen. Acylierungen, Aminosäureaustausche sowie Fusion mit Proteinen oder anderen Molekülen ein. Solche Modifikationen können z.B. durch Rekombination und/oder Expression und/oder chemische und/oder enzymatische Modifikation einzelner oder mehrerer Aminosäuren erzielt werden.

Das Protein gemäß Seq ID No.2 weist eine Vielzahl von Vorteilen auf. Es kann, besser als das Wildtyp-CGB-Protein oder Varianten davon mit 80% oder mehr Homologie, prokaryonte DNA über nicht methylierte CpG-Motive binden. Dadurch wird es möglich, aus einem Gemisch von prokaryonter und eukaryonter DNA die prokaryonte DNA spezifisch abzutrennen und/oder anzureichern. Dies ermöglicht letztendlich einen schnellen und einfachen Erregemachweis sowie eine frühzeitige Diagnose von Infektionen, die durch bakterielle Erreger verursacht sein können. Vice versa kann die Erfindung auch zur Abreicherung mikrobieller DNA im Sinne einer Reinigung bei klinischen Zuständen angewandt werden, die mit einem unphysiologischen Vorkommen von Bakterien oder deren Spaltprodukten Körperflüssigkeiten, insbesondere Blut, von Patienten einhergehen. Dies gilt umso mehr, da gut belegt ist, daß Bakterien aber auch deren Spaltprodukte, wie beispielsweise bakterielle DNA, für eine Vielzahl den Patienten schädigenden biologischen Effekte verantwortlich sind.

Aufgrund der guten Bindungsfähigkeit des Proteins gemäß Seq ID No.2 an nicht methylierte CpG-Motive prokaryonter DNA ist Gegenstand der Erfindung ein Verfahren zur Trennung und/oder Anreicherung prokaryonter DNA mit den Schritten:
a) Kontaktieren mindestens einer in Lösung befindlichen prokaryontischen DNA mit einem spezifisch prokaryontische DNA bindenden Protein, das eine Aminosäuresequenz gemäß SEQ ID NO: 2 aufweist, wodurch ein Protein-DNA-Komplex gebildet wird, wobei das Protein in der Lage ist, nicht-methylierte CpG-Motive zu erkennen, und
b) Separation des Komplexes.

Weiterhin ist es bevorzugt, dass nach Schritt b) als Schritt c) die prokaryontische DNA amplifiziert wird. Ein derartiges Verfahren umfasst bevorzugt die Schritte:
a) Isolierung der prokaryontischen DNA aus dem Protein-DNA-Komplex,
b) Denaturierung der doppelsträngigen DNA,
c) Hybridisierung der Einzelstränge der DNA, mit komplementären Primern,
d) Generierung von Doppelstrangfragmenten über eine Reaktion mit Polymerasen, und
e) Wiederholung dieser Schritte zum gewünschten Amplifikationsgrad.

Es ist bevorzugt, dass das Verfahren weiterhin umfasst:
a) Klonierung der isolierten prokaryontischen DNA-Sequenzen in Vektoren,
b) Transformation geeigneter Wirtszellen mit diesen Vektoren,
c) Kultivieren dieser transformierten Zellen,
d) Isolation der Vektoren aus diesen Zellen, und
e) Isolierung der DNA.

Die DNA kann aufgereinigt und wieder in Lösung gebracht sein oder direkt in der Ursprungsquelle (z. B. Körperflüssigkeit, wie Blut, Serum, Trachealaspirat, Urin, bronchalveoläre Lavage, Nasenabstrich, Hautabstrich, Punktionsflüssigkeit) vorliegen.

Die Separation kann mittels verschiedener Verfahren zur Trennung, Isolierung oder Anreicherung von DNA-Protein-Komplexen oder DNA-Polypeptid-Komplexe erfolgen, die dem Fachmann hinlänglich bekannt sind. Dabei werden bevorzugt Methoden angewendet, bei denen das DNA-bindende Protein an einen Träger immobilisiert ist oder wird, um die DNA aus der Probelösung zu trennen und/oder anzureichern.

Gemäß einer bevorzugten Ausführungsform schließt sich an die Separation ein Schritt zur Abtrennung der DNA vom Protein aus dem Komplex an. Dies kann beispielsweise durch herkömmliche Verfahren zur DNA-Aufreinigung erfolgen, die dem Fachmann bekannt sind. Im einfachsten Falle beruht die Abtrennung auf der Änderung des pH-Wertes oder der Salzkonzentration (z. B. auf 1 M NaCl) des Mediums/Puffers oder der Zufügung chaotroper Reagenzien, etc; also geeignete Parameter, die zur Auflösung des Protein-DNA-Kornplexes führen Solche Methoden sind dem Fachmann bekannt.

Gemäß einer weiteren bevorzugten Ausführungsform ist das Protein an einen Träger gebunden. Diese Ausführungsform stellt eine besonders einfache Möglichkeit der Anreicherung prokaryonter DNA dar, da die Separation aus der Lösung besonders einfach, beispielsweise durch physikalischer Entfernung (z.B. Abzentrifugation) des oder der beladenen Träger aus der Lösung, erfolgen kann.

Für die Lösung für die prokaryonte DNA kommt grundsätzlich jedes geeignete Lösungsmittel in Frage. Besonders zweckmäßig ist das Verfahren jedoch zur Anreicherung prokaryonter DNA aus Lösungen, die verschiedene biomolekulare Spezies, insbesondere verschieden Arten von DNA, enthalten. Die Erfindung betrifft vorzugsweise ein Verfahren zur Trennung und

Anreicherung prokaryonter oder viraler DNA aus einem Gemisch von prokaryonter und eukaryonter DNA. Dabei wird beispielsweise die in Körperflüssigkeiten befindliche prokaryonte DNA durch spezifische Bindung an das Protein gemäß Seq. ID No.2 von der eukaryonten DNA getrennt und angereichert. Die so angereicherte prokaryonte DNA erleichtert den Nachweis prokaryonter Erreger mit Hilfe molekularbiologischer Methoden und kann zur Diagnose von Krankheiten, die durch pathogene Erreger verursacht werden, beitragen.

Insbesondere die Ausführungsform, bei der das DNA-bindende Protein gemäß SEQ ID No.2 an die Oberfläche eines Trägers immobilisiert ist, eignet sich für eine Adsorption prokaryonter DNA aus Körperflüssigkeiten, vorzugsweise aus dem Blut. Dieser Ansatz bietet überdies die Möglichkeit mikrobielle DNA, die im Blut oder anderen Körperflüssigkeiten vorliegt, aus diesen zu entfernen. Die so von der mikrobiellen DNA, die auch allein in der Lage ist, schwere Entzündungsreaktionen bei Patienten auszulösen, gereinigte Körperflüssigkeit (z. B. Vollblut, Serum oder Liquor), kann dann in den Körper zurückgeführt werden. Dieses Prinzip kann also zur Abreicherung prokaryonter DNA aus physiologischen Flüssigkeiten im Sinne der Reinigung eingesetzt werden, wobei die speziellen Bindungseigenschaften des Proteins gemäß SEQ ID No.2 genutzt werden.

Zur Steigerung der Bindungskapazität und -effizienz für die nicht-mehtylierten CpG-Motive einer DNA stellt die Erfindung ein Verfahren zur Verfügung, dass die Bindungskapazität und -effizienz des Proteins erhöht und somit eine verbesserte Trennung und/oder Anreicherung nicht-methylierter DNA aus einem Gemisch von methylierter DNA und nicht-methylierter DNA ermöglicht.

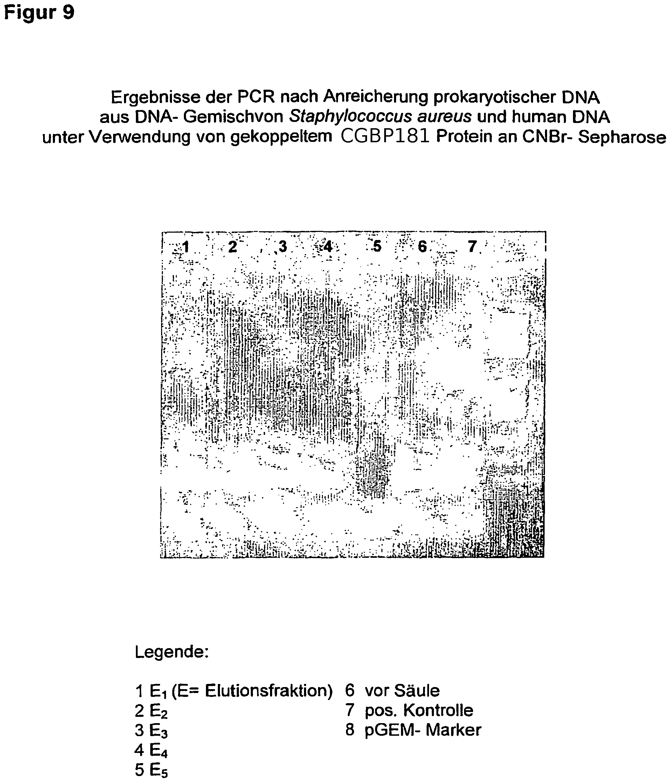

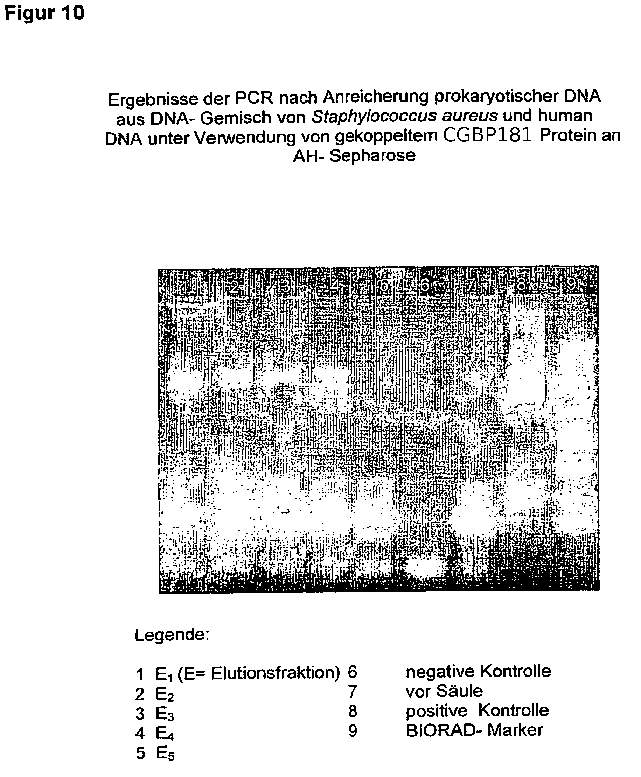

Erfindungsgemäß wird dies durch die indirekte Kopplung des Proteins an die Matrix erreicht. Dieses Verfahren wird nachfolgend unter Bezugnahme auf die Figuren 9 und 10 beschrieben.

Zur Steigerung, der Bindungskapazität und -effizienz des CGBP181 Proteins für nicht-methylierte CpG-Motive enthaltende DNA erfolgt die indirekte Bindung des Proteins an die Matrix über einen Spacer. Durch die Kopplung des Proteins über einen Spacer an die Matrix wird der Grad der Beweglichkeit sowie die Anzahl der freien Bindungsstellen des CGBP181-Proteins erhöht. Dadurch wird eine Steigerung der Bindungskapazität- und effizienz erreicht. Weiterhin kann dadurch die Menge des eingesetzten Proteins reduziert werden.

Als Spacer im Sinne dieser Erfindung werden kurze kettenartige Moleküle verstanden, welche einen räumlichen Abstand zwischen Matrix und dem erfindungsgemäß eingesetzten Protein, z. B. CGBP181-Protein, ermöglicht Solche Spacer sind dem Fachmann bekannt, z. B. von der Affinitätschromatographie oder der Immobilisierung von Proteinen. Solche kettenartige Moleküle sind aus C- und H-Atomen sowie ggf. Heteroatomen, z. B. N, aufgebaut. Diese kettenartigen Moleküle sind aus einzelnen Kettelgliedern auf der Basis der C-Atome z. B. CH₂ und ggf. vorhandenen Heteroatomen, z. B. NH aufgebaut. Der Spacer weist insbesondere 4 bis 20, vorzugsweise 7 bis 10, Kettenglieder auf. Ein besonders bevorzugter Spacer leitet sich von Diaminhexan (NH₂-(CH₂)₆-NH₂) ab. Antikörper sind im Sinne der vorliegenden Erfindung nicht als Spacer zu betrachten.

Als Matrix im Sinne dieser Erfindung werden Substanzen bezeichnet, die als Träger für Spacer und Protein fungieren. Trägermaterialien können beispielsweise Sepharose, Perizellulose, Silica o. ä. dem Fachmann bekannte Substanzen sein.

Als Körperflüssigkeiten im Sinne der Erfindung werden alle vom Körper eines Säugers, einschließlich Mensch, stammenden Flüssigkeiten verstanden, insbesondere solche in denen Krankheitserreger vorkommen können, wie z. B. Blut, Urin, Liquor, Pleural-, Perikardial-, Peritoneal- sowie Synovialflüssigkeit. Die auf humanes Blut bezogene Beschreibung der Erfindung stellt keine Einschränkung sondern nur eine beispielhafte Anwendung dar.

Unter bakteriellen Erregern werden vorzugsweise Erreger einer Sepsis, aber auch alle anderen bakteriellen Erreger von Infektionen verstanden. Sie können sich dabei von kommensalen Erregern unterscheiden, die zur normalen Besiedlung des Organismus gerechnet werden und gelegentlich auch in Untersuchungsproben von Patienten gefunden werden, aber keine klinische Bedeutung haben.

Bei der Isolierung der Gesamt-DNA aus infizierten Körperflüssigkeiten kann das Verhältnis Wirts-DNA zur Erreger-DNA oft nur 1:10⁻⁶ bis 1:10⁻⁸ oder sogar noch weniger betragen. Das erfindungsgemäße Verfahren ermöglicht durch die spezifische Bindung prokaryonter DNA an das Protein gemäß SEQ ID No.2 eine Anreicherung um 1 Potenzeinheit und mehr.

Das Protein gemäß SEQ ID No.2 kann direkt oder indirekt an den Träger gekoppelt sein. Die Art der Kopplung hängt von dem Träger und dem Trägermaterial ab. Als Träger kommen dabei insbesondere Membranen, Mikropartikel und Harze oder ähnliche Materialien für Affinitätsmatrices in Frage. Geeignete Materialien zur Anbindung des Proteins gemäß SEQ ID No.2, sowie - abhängig von der Art des Materials - die Durchführung der Anbindung, sind dem Fachmann hinlänglich bekannt. Für die indirekte Kopplung eignen sich beispielsweise spezifische Antikörper gegen das Protein gemäß Seq ID No.2 oder das Polypeptid, die ihrerseits durch bekannte Verfahren an den Träger gebunden sind.

Eine Anwendung des erfindungsgemäßen Verfahrens besteht in der Anreicherung prokaryonter DNA. Eine weitere Anwendung besteht in der Trennung von prokaryonter DNA aus einem Gemisch eukaryonter und prokaryonter DNA durch die Bindung der prokaryonten DNA an das Protein gemäß SEQ ID No.2, welches z.B. an eine Matrix immobilisiert wurde. Das Gemisch aus körpereigner und prokaryonter DNA wird mittels geeigneter Verfahren mit der Affinitätsmatrix in Verbindung gebracht, und dabei wird die prokaryonte DNA an das immobilisierte Protein gebunden; die eukaryonte DNA durchläuft zum Beispiel eine Trennsäule und kann separat gesammelt werden. Affinitätsmatrices können beispielsweise polymere Polysaccharide, wie Agarosen, andere Biopolymere, synthetische Polymere, oder Träger mit Silikat-Grundgerüst, wie poröse Gläser oder sonstige feste oder flexible Träger, sein, an welchen das DNA-Bindende Protein gemäß SEQ ID No.2 immobilisiert wird. Nach erfolgter Trennung prokaryonter von eukaryonter DNA wird die Affinitätsmatrix mit einem geeigneten Reagenz gespült, so daß entweder das Bindungsprotein mit der gekoppelten prokaryonten DNA von der Matrix und/oder die prokaryonte DNA von dem Bindungsprotein getrennt wird und für weitere Arbeitsschritte in ausreichender Menge zur Verfügung steht.

Eine weitere Anwendung des erfindungsgemäßen Verfahrens besteht in der Trennung und Anreicherung prokaryonter DNA von eukaryonter DNA durch Bindung der prokaryonten DNA an das Protein gemäß SEQ ID No.2 welches an Milkropartikeln immobilisiert wurde. Hierbei kommen alle Mikropartikel in Frage, die eine Immobilisierung des DNA-bindenden Proteins ermöglichen. Solche Mikropartikel können aus Latex, Kunststoff (z. B. Styropor, Polymer), Metall oder ferromagnetischen Stoffen bestehen. Weiterhin können auch fluoreszierende Mikropartikel, wie sie beispielsweise von der Firma Luminex angeboten werden, verwendet werden. Nachdem die prokaryonte DNA an die an Mikropartikel immobilisierten Proteine gebunden wurde, werden die Mikropartikel mit geeigneten Methoden, wie beispielsweise Filtration, Zentrifugation, Fällung, Sortierung über Messung der Fluoreszensintensität oder magnetische Verfahren, von dem Stoffgemisch getrennt. Die prokaryonte DNA steht nach Trennung von den Mikropartikeln zur weiteren Verarbeitung zur Verfügung.

Eine andere Anwendung des erfindungsgemäßen Verfahrens besteht in der Trennung und Anreicherung prokaryonter DNA von eukaryonter DNA durch Bindung der prokaryonten DNA an das Protein gemäß SEQ ID No.2, welches anschließend durch Elektrophorese von übrigen Bestandteilen des Gemisches getrennt wird.

Eine weitere Anwendung des erfindungsgemäßen Verfahrens besteht in der Trennung und Anreicherung prokaryonter DNA von eukaryonter DNA durch Bindung der prokaryonten DNA an das Protein gemäß SEQ ID No.2, wobei das Protein anschließend an entsprechende Antikörper gebunden wird. Die Antikörper können an feste oder flexible Substrate, z. B. Glas, Kunststoffe, Silizium, Mikropartikel, Membranen gebunden sein, oder sich in Lösung befinden. Nach Bindung der prokaryonten DNA an das Protein und dessen Bindung an den spezifischen Antikörper erfolgt die Trennung aus dem Stoffgemisch mit dem Fachmann bekannten Methoden.

Das erfindungsgemäße Verfahren kann auch zur Reinigung von Körperflüssigkeiten von prokaryonter DNA eingesetzt werden. Hierbei ist es zweckmäßig, daß die Separation extrakorporal unter sterilen Bedingungen erfolgt, damit die Körperflüssigkeiten wieder in den Körper zurückgeführt werden können, so daß das körpereigene Immunsystem bei der Beseitigung von Infektionen unterstützt wird, indem die sich in den Körperflüssigkeiten befindende prokaryonte DNA entfernt wird.

Bei der extrakorporalen Entfernung der prokaryonten DNA aus Körperflüssigkeiten kommen alle geeigneten chemischen, mechanischen oder elektrochemischen Verfahren in Betracht. Weiterhin stellt auch die Kombination mit anderen extrakorporalen Verfahren, wie Hämoperfusion, Herz-Lungen-Maschine oder Endotoxin-Adsorber, eine weitere zweckmäßige Anwendung dar.

Das Protein gemäß SEQ ID No.2 kann auch zur Detektion prokaryonter DNA eingesetzt werden. Hierbei schließt sich nach der Anreicherung der prokaryonten DNA ein Schritt zur Amplifikation der prokaryonten DNA an, wozu sich alle gängigen Amplifikationsmethoden eignen (PCR, LCR,LM-PCR etc.).

Das erfindungsgemäße Verfahren, insbesondere mit den vorstehend beschriebenen Ausführungsformen hat den Vorteil, daß durch spezifische Bindung nicht-methylierter CpGmotiv-reicher prokaryonter DNA an Proteine mit spezifischer Affinität für solche Strukturen eine Konzentrierung prokaryonter DNA aus der Gesamt-DNA eines infizierten Wirts gelingt und damit die Nachweisempfindlichkeit für Verfahren zum Nachweis von Erreger-DNA in Körperflüssigkeiten stark erhöht wird.

Die Abtrennungsmöglichkeiten prokaryonter DNA von eukaryonter DNA mit einem spezifisch bindenden Protein sind nicht zeitaufwendiger als bekannte Methoden zur Isolierung von Gesamt-DNA. Der nachfolgende DNA-Nachweis kann über eine PCR-Reaktion erfolgen. Eine nested PCR wird in den meisten Fällen nicht notwendig sein, so daß eine beträchtliche Zeitersparnis in der Diagnostik möglich wird.

Bereits vorstehend wurde angesprochen, das Protein gemäß SEQ ID No.2 zur Abreicherung prokaryontischer DNA in physiologischen Körperflüssigkeiten einzusetzen. Abreicherung im Sinne der vorliegenden Erfindung bedeutet dabei, daß die Menge der prokaryontischen DNA verringert wird. Diese Möglichkeit zur Verringerung der prokaryontischen DNA ermöglicht es auch, die Proteine in der Umwelttechnik, der Abwasserwirtschaft und der Klimatechnik einzusetzen

Die Erfindung betrifft außerdem ein Verfahren zur Abtrennung und Anreicherung von nichtmethylierte genomischer DNA aus einem Gemisch von nicht-methylierter genomischer und methylierter genomischer DNA. Durch die Bindung der nicht-methylierten genomischen DNA an das an eine Matrix gekoppeite CGBP181 Protein wird die methylierte genomische DNA abgetrennt. Diese Verfahrensweise trägt wesentlich zur vereinfachten Untersuchung der Methylierungsmuster von methylierter genomischer DNA bei und ermöglicht die Diagnose von Krankheiten, die ein spezifisches Methylierungsmuster aufweisen, insbesondere Krebserkrankungen.

Das Verfahren zur Abtrennung und/oder Anreicherung von nicht-methylierter genomischer DNA aus einem Gemisch von nicht-methylierter genomischer und methylierter genomischer DNA umfasst die Schritte:
a) Kontaktieren einer in Lösung befindlichen nicht-methylierten genomischen DNA mit einem spezifisch nicht-methylierte DNA bindenden Protein, das eine Aminosäuresequenz gemäß SEQ ID NO: 2 aufweist, wodurch ein Protein-DNA-Komplex gebildet wird, und
b) Separation des Komplexes.

Die Erfindung betrifft darüber hinaus die Verwendung eines Kit zur Anreicherung prokaryonter DNA mittels eines der vorstehend beschriebenen Verfahren, in dem zumindest das Protein gemäß SEQ ID No.2 gegebenenfalls zusammen mit weiteren geeigneten Reagenzien zur Verfahrensdurchführung enthalten sind.

Der Kit kann neben dem Protein gemäß SEQ ID No.2 mindestens ein Set von Primem, welche zur Amplifikation genomischer DNA bestimmter Prokaryonten unter Standardbedingungen geeignet sind, enthalten.

Die Erfindung wird nachfolgend anhand der Beispiele noch näher erläutert, ohne sie aber darauf einzuschränken.

### Beispiel 1: Herstellung des Proteins gemäß SEQ ID No.2:

Aus der DNA Sequenz für das komplette CGB- Protein wurden die Primer 1 (GGATCCGGTGGAGGGCGCAAGAGGCCTG -fw SEQ ID Nr. 3) und 2 (AAGCTTAGAGGTAGGTCCTCAT-CTGAG-rv SEQ-ID Nr. 4) konstruiert, die ein verkürztes DNA-Fragment amplifizieren, das für ein verkürztes CPG-bindendes Protein, CGBP181. kodiert Das DNA Fragment wurde nach Spaltung mit den Restriktionsenzymen *BamHI* und *Hind III* in den Vektor pQE9 (Qiagen) ligiert In pQE9 entsteht ein offener Leserahmen, in dem an das 5' Ende ein für 6 x His-Tag kodierendes DNA Fragment fusioniert wird (pQE9[6HisCGBP181]). Die vollständige Aminosäuresequenz des kodierenden Fusionsproteins 6HisCGBP181 ist nachfolgend gezeigt, wobei der fett gedruckte Abschnitt das Peptid CGBP181 repräsentiert und die schräggedruckten Abschnitte fusionierte Fremdaminosäuren vom Plasmid pQE9 anzeigen.

Das Plasmid pQE9[6HisCGBP181] wurde in den *E. c*oli Expressionsstamm M15[pREP4] (Qiagen) transformiert. Der Klon wird im weiteren M15[pCGBP181] bezeichnet und das exprimierte Protein rCGBP181. Die Expression des Proteins rCGBP181 erfolgte nach folgendem Protokoll: Eine Kolonie des Expressionsstammes M15[pCGBP181] wird in 2 ml Luria Medium mit 100µg/ml Ampicillin und 25µg/ml Kanamycin bei 37°C unter Schütteln über Nacht angezüchtet. Anschließend wird die Vorkultur in 200 ml vorgewärmtes Nährmedium, das die gleichen Antibiotikakonzentrationen enthält, überführt. Nach 3 Stunden Wachstum bei 37°C unter Schütteln wird IPTG zur Induktion der Expression zugegeben und weitere 5 Stunden inkubiert. Danach werden die Bakterien abzentrifugiert und das Sediment in 5 ml 0.2 M Trispuffer, pH 7.5 resuspendiert. Die Bakterien werden im Eisbad 5 x 1 min mit Ultraschall behandelt. Nach der Zentrifugation wird das Sediment in 10 ml 0.2 M Tris, 2M Harnstoff, pH7.5 resuspendiert und 15 min geschüttelt. Nach erfolgter Zentrifugation wird das verbliebene Sediment in 0.2 M Tris, 6M Guanidinhydrochlorid, 0.001 M Dithioeritrit (DTE), 0.02 M Imidazol aufgenommen und suspendiert. Die Inclusionbodies werden unter Agitation für 1 Stunde bei Raumtemperatur gelöst Nach Zentrifugation befindet sich das Rohprotein im Überstand und kann direkt auf eine 3 ml Ni-Agarosesäule aufgetragen werden. Die nächsten Schritte sollten im Kühlraum bei +4 bis +6°C erfolgen. Zunächst wird die Säule mit 0.2 M Tris, 6M Guanidinhydrochlorid, 0.001 M Dithioeritrit (DTE), 0.02 M Imidazol Puffer, pH7.5 gewaschen bis die Extinktion die Nulllinie erreicht hat. Von hier aus kann rCGBP181 auf verschiedenen Wegen gewonnen werden: 1. Als denaturiertes Protein gelöst in 6M Guanidinhydrochlorid oder 6 M Harnstoff und 2. als natives Protein löslich in Puffern physiologischer Konzentration. Im 2. Fall ist die Ausbeute aber geringer.

### Reinigung nach Methode 1 (denaturiert):

Das Protein rCGBP181 wird von der Ni-NTA Agarose mit einem Imidazolgradienten von 0 - 0.5 M eluiert M in dem Puffer 0.2 M Tris, 6M Guanidinhydrochlorid, 0.001 M Dithioeritrit (DTE), 0.02 M Imidazol, pH7.5 als Grundlage. Dabei wird rCPGbP181 bei 0.2 - 0.3 M Imidazol von der Säule abgelöst. Das so gewonnene Protein wird gegen 0.2 M Tris, 6M Harnstoff. 0.001 M Dithioeritrit (DTE), pH7.5 dialysiert und eingefroren. Bei Dialyse gegen physiologische Puffer fällt so gereinigtes rCGBP181 aus.

### Reinigung nach Methode 2 (nativ):

Nach dieser Methode wird die Guanidinhydrochtorid Konzentration von 6 molar auf der Ni-NTA Agarose mit dem gebundenem rCGBP181 über einen Gradienten auf 0 molar Guanidinhydrochlorid gebracht. Grundlage ist der Puffer 0.2 M .Tris, 0.5 M NaCl, 0.001 M Dithioeritrit (DTE), 0.02 M Imidazol, pH7.5. Hier wählten wir die Flussgeschwindigkeit von 0.5 ml/min. Danach wurde zur Elution ein Imidazolgradient von 0 bis 0.5 molar angelegt in Puffer 0.2 M Tris, 0.5 M NaCl, 0.001 M Dithioeritrit (DTE), pH7.5 als Grundlage. Auch hier wurde ein wesentlicher Anteil des gebundenen Proteins (20%) bei 0.2 bis 0.3 molar Imidazol eluiert. Dieses native rCGBP181-Eluat blieb in diesem Puffer gelöst, auch nach Dialyse in PBS. Von Nachteil ist aber, dass ca. 80% des an Ni-NTA Agarose gebundenen rCGBP181 unter diesen bedingungen auf der Säule verblieben und nachträglich nur unter den denaturierenden Bedingungen von Methode 1 noch gewonnen werden konnten. Das heißt, die Ausbeute der verwendeten Methode 2 ergab nur 20% natives, in physiologischen Puffern lösliches rCGBP181.

### Beispiel 2: Erregernachweis mittels nested PCR:

Frisches, heparinisiertes Humanblut, das *Streptococcus pyogenes* mit 10³ /ml koloniebildende Einheiten als Erreger enthält, wird für den Erregemachweis verwendet. Die DNA wird mittels Absorption an DNA bindende Matrix mit kommerziellen Kits zur Isolierung von Gesamt-DNA aus Körperflüssigkeiten nach abgewandelter Vorschrift der Hersteller isoliert. Dazu werden 100 µl infiziertes Blut in Eppendorf Tubes mit 200 µl des Totallysispuffers versetzt, der Proteinase K und SDS enthält. Das Gemisch wird 30 min bei 37°C inkubiert, und danach 20 min auf 95°C erhitzt. Nach dem Abkühlen werden 20 µg Mutanolysin zugegeben und weitere 60 min bei 37°C inkubiert Nach Zentrifugation wird das Gemisch auf die Zentrifugationssäulchen mit DNAbindender Matrix aufgetragen und die DNA nach Vorschrift des Hersteilers gereinigt. Die gereinigte DNA wird in einem Endvolumen von. 100 µl 0.01 molar Trispuffer, pH 7.5 oder in gleicher Menge Elutionpuffer des Herstellers aufgenommen. Für den Erregernachweis wurden Primer zur Identifizierung des Streptolysin O Gens (slo) konstruiert.

### 1. PCR. Amplification eines 465 bp Fragmentes

Forward-Primer 1: 5'-AGCATACAAGCAAATTTTTTACACCG
Reverse-Primer 2: 5'- GTTCTGTTATTGACACCCGCAATT
Primer Konzentration 1 mg/ml
Ansatz:
   5µl DNA-Isolat
   0.5 µl Primer fw 1
   0.5µl Primer rv 2
   14µl Aqua dest
   total 25 µl in Ready to go Kit (Amersham-Pharmacia)

### Reaktion:

5 min 95°C
Zyklen 40 (30 sec. 95°C, 30 sec 51 °C, 3 min 72°C, 1 x 7min 72°C)

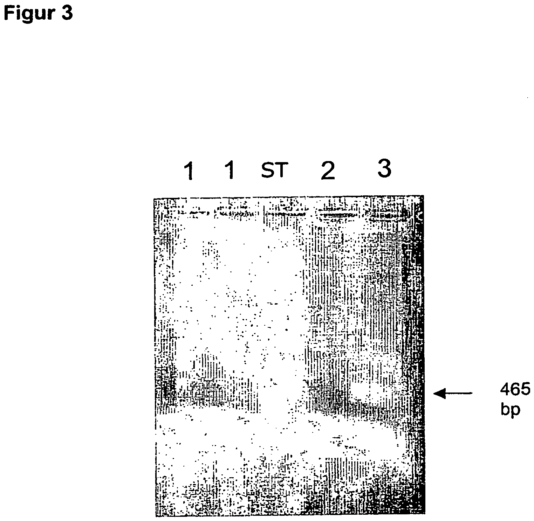

in Figur 3 ist 1) PCR von Streptokokken-DNA in Humanblut dargestellt (je 10 µl des 25 µl Ansatzes äufgetrennt: 1)PCR Ansatz mit 5 µl Template DNA; 2) Ansatz mit 5µl Template, 1:10 verdünnt. 3) Positivkontrolle: 0.2 µl Streptokokken-DNA als Template ohne Anwesenheit eukaryotischer DNA aus Blut. ST) Molekulargewichtsstandard).

Ergebnis: Die 1. Primär PCR ergibt keine positive Reaktion. Deshalb wurde nachfolgend eine 2. PCR (nested PCR) durchgeführt.

### 2: PCR, nested PCR. Amplifikation eines 348 bp Fragmentes innerhalb des obigen slo-Fragments

Forward Primer 3: 5'- CCTTCCTAATAATCCTGCGGATGT
Reverse Primer 4: 5'- CTGAAGGTAGCATTA/G TCTTTGATAACG
Primer-Konzentration: 1mg/ml
Ansatz:
   5µl aus PCR1, Probe 1, Abb. 1
   0.5 µl Primer fw 1
   0.5µl Primer rv 2
   14µl Aqua dest
   total 25 µl in Ready to go Kit (Amersham-Pharmacia)

### Reaktion:

5 min 95 °C
Zyklen 40 (30 sec. 95°C, 30 sec 54° C, 3 min 72°C, 1 x 7 min 72°C)

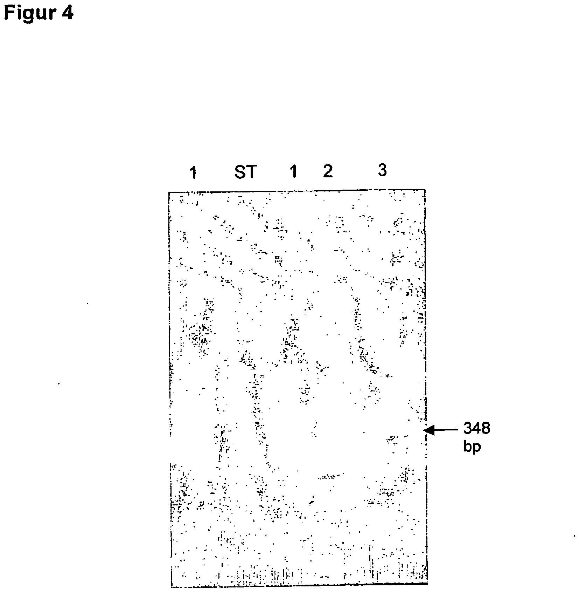

In Figur 4 ist die nested PCR mit den PCR-Produkten aus dem Primär PCR-Ansatz in Figur 3 als Template gezeigt. Die Proben entsprechen denen aus Figur 3.

Ergebnis: In der nested PCR wird das gewünschte slo-DNA, Fragment amplifiziert bei einer Konzentration von 100 Streptokokken zellen pro 100 µl Blut (Probe 1). Das entspricht bei 5µl Einsatz in der 1. PCR (Fig. 3) ca 5 bis 10 Templates. Bei einer 1:10 Verdünnung (Probe 2) ist die Empfindlichkeit erschöpft (0,5 bis 1 Template).

Aus diesen Versuchen ist ersichtlich, dass für einen erfolgreichen PCR-Nachweis von Erregern im Blut die Gesamt-DNA aus mindestens 1 bis 5 ml Blut isoliert werden muss. Die Gesamt-DNA-Konzentration ist dann aber zu groß, um dann direkt in einer PCR eingesetzt zu werden.

Andere erregerspezifische Nukleinsäurenachweise ohne Amplifikationsschritt durch direkte Detektion der bakteriellen DNA z.B. mittels DNA Hybridisierung sind ebenfalls zu unempfindlich" was vor allem am hohen Überschuss von humaner DNA gegenüber bakterieller DNA liegt. Hierbei sind zudem kompetitive Prozesse bei der DNA-Analyse sowie die geringe Menge an bakterieller DNA als hinderlich für eine qualitative und quantitative Analyse anzusehen. Die üblichen Methoden zur DNA-Isolierung reichem die Gesamt-DNA einer Körperflüssigkeit an, sodass das Verhältnis Wirts-DNA zu mikrobieller DNA zwischen 1 : 10⁻⁶ und 1 : 10⁻⁸ betragen kann. Aus diesem Unterschied ist die Schwierigkeit des Nachweises mikrobieller DNA in Körperflüssigkeiten gut nachzuvollziehen.

### Beispiel 3: Ermittlung der Bindungseigenschaften von rCGBP181:

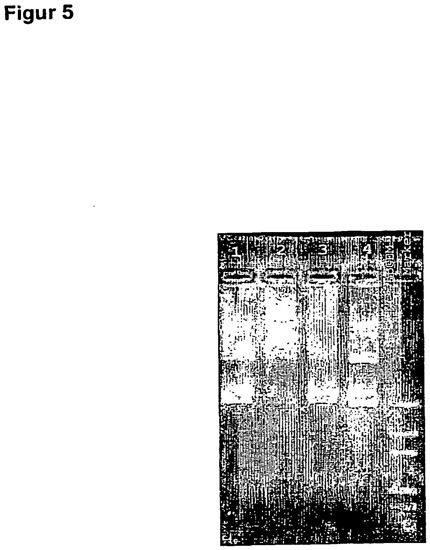

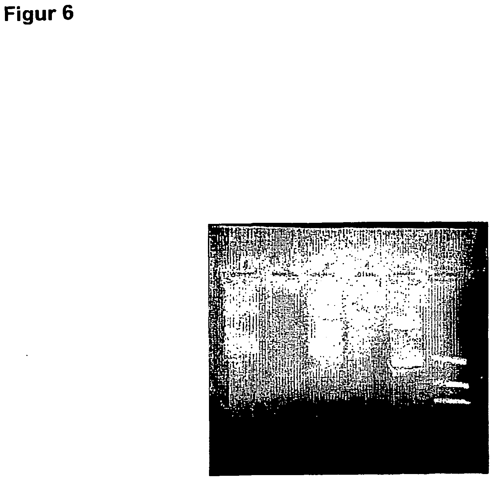

In Getretardierungsexpenmenten wurde sowohl die Bindung des denaturierten als auch die des nativen Proteins rCGBP181 an methylierte und nicht-methytierte DNA-Moleküle mit CpG Motiven untersucht. Als Test DNA wurde das *E. coli* Plasmid pUC18 verwendet mit einem inserierten M-Proteingensegment von *Streptococcus dysgalactiae supsp. equisimilis* (Geyer et. al. FEMS Immunol. Med. Microbiol. 26:11-24, 1999). Die Plasmidpräparation wurde geteilt und die eine Hälfte mit dem CpG-Methylase Kit von New England BioLabs methytiert. Beide Präparationen wurden mit rCGBP181 (nativ oder denaturiert) gemischt und im Agarosegel elektrophoretisch aufgetrennt. Die Ergebnisse sind in den Figuren 5 und 6 einzusehen. Sowohl rCGBP181 in nativer als auch in denaturierter Form zeigte höhere Affinität zur nichtmethylierten Plasmid DNA, was die selektive Bindungseigenschaft für nichtmethyliete CpG-reiche DNA bestätigt.

Beschreibung des Gelretardierungsexperiments gemäß Figur 5: Je 5 µl (72 ng) methylierte und 1 µl (142 ng) nicht methylierte pUC18*emm* DNA wurden mit 5 µl (0.5 µg) nativem rCGBP181 gemischt und auf ein Volumen von 35 µl mit dem Puffer. 0.01 M Tris, 0.08M NaCl, 0.001 M EDTA, 0.005M DTE, 5% Glycerin, pH7.8 aufgefüllt. Nach 30 min Inkubation bei 20°C wurden die Gemische elektrophoretisch in 1.5%iger Agarose aufgetrennt. In Lanes 1 und 3 wurde methylierte DNA und in Lanes 2 und 4 nichtmethylierte DNA aufgetragen. In Lanes 1 und 2 wurde die DNA mit nativem rCGBP181 gemischt. Lane 2 zeigt, dass nichtmethytiertes pUC18*emm* mit rCGBP181 interagiert, keine Wechselwirkung zeigte dagegen rCGBP181 mit methyliertem pUCi8*emm* (Lane1). Lanes 4 und 5 sind die Plasmide ohne Zusatz von rCGBP181 als Kontrollen.

Beschreibung des Gelretardierungsexperiment aus Figur 6 von nicht methyliertem und methyliertem pUC18*emm* nach Inkubation mit denaturiertem rCGBP181. Die Konzentrationen entsprechen denen von Figur 5. In Lanes 1 und 3 wurde methylierte DNA und in Lanes 2 und 4 nichtmethyllette DNA aufgetragen. In Lanes 1 bis 4 wurde die DNA mit zwei verschiedenen Chargen von denaturiertem rCPGbP181 gemischt Lanes 2 und 4 zeigen, dass nichtmethytiertes pUC18*emm* auch mit denaturiertem rCPGbP181 interagiert, keine Wechselwirkung zeigte dagegen rCGBP181 mit methyliertem pUC18*emm* (Laries 1 und 3). Lane 5 pUC18*emm* rCGBP181 als Kontrolle.

### Beispiel 4: Bindung und Trennung eines Gemisches von Kalbsthymus-DNA und bakterieller DNA an immobilisiertes CGBP181.

Gereinigtes CGBP181 wurde mittels Glutaraldehyd an Aminahexyl-Sepharose (Amersham-Biosciences) gekoppelt nach der Vorschrift von Cambiasso et al. (Cambiasso, C. et al., Immunochemistry 12:273-278, 1975) gekoppelt. Die immobilisierte Proteinkonzentration betrug 0.3 mg pro Milliliter Sepharose. 300 µl Sepharose wurden in ein Spin-Filter Röhrchen gegeben mit innertem Frittenmaterial, das weder DNA noch Protein absorbiert, aber die Sepharose zurückhält.

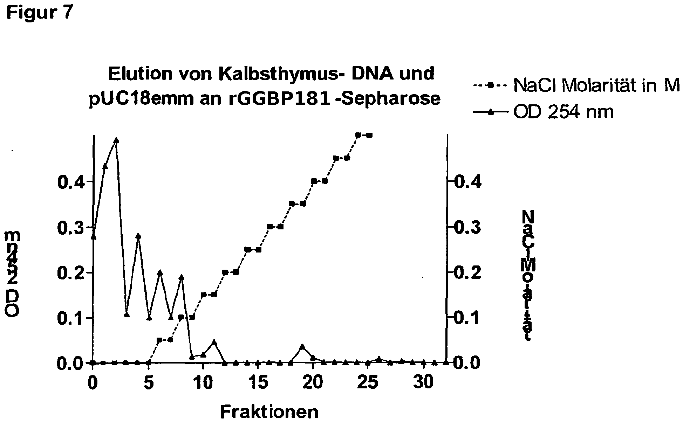

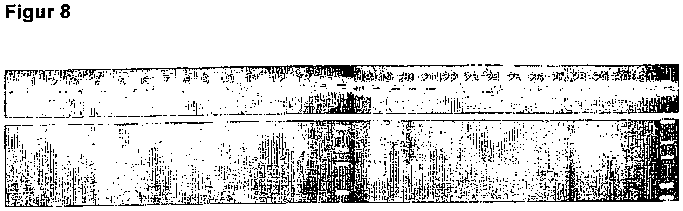

200 ng Kalbsthymus-DNA und 25 ng pUC18*emm* wurden in 100 µl 20 mM Tris-HCL Puffer, pH 7.5 gelöst und auf das so präparierte Säulchen gegeben. Nach jedem Schritt wurde die Flüssiskeit 0,5 min bei 14 000 RPM in einer Eppendorfzentrifuge in je ein frischen Eppendorfröhrchen zentrifugiert. So wurde in zweier Schritten die NaCl-Konzentration erhöht von 0 auf 1M jedem Röhrchen wurde eine DNA Fallung durchgehürt indem 10 µl 4 M Acetat, pH4,5 und 250 µl abs. Ethanol zugegeben wurden, gemischt und 15 min bei 14 000 RPM zentrifugiert. Danach wurde der Überstand abgegossen und das Präzipitat mit 300 µl 70%igem Ethanol gewaschen. Nach Abgießen wurde der Rückstand 5 'min in einer vakuumzentrifuge getrocknet und anschließend in 15 µl dest. Wasser (PCR-tauglich) aufgenommen. Zum einen wurde die Extinktion bei 254 nm von je 10 µl der Proben gemessen (Figur 7). Zum anderen wurde mit je 3 µl jeder Probe eine PCR mit Sequenzprimem für PUC18 durchgeführt (Figur 8).

Das Ergebnis (Figur 7, 8) zeigt daß die eukaryotische Kalbsthymus-DNA am Anfang zwischen 0 bis 0,1 M NaCl von der Säule gewaschen wird, während die prokaryotische DNA (pUC18*emm*) in der Fraktion bei 0.3 M NaCl eluiert wurde. Das zeigt, dass eukaryotische DNA eine niedrigere Affinität zu CGBP181 aufweist und somit eine eindeutige Trennung beider DNA-Fraktionen erreicht wurde.

### Beispiel 5: Steigerung der Sindungseigenschaften des CGBP181 Proteins, die aus der indirekten Bindung dieses Proteins über einen Spacer an eine Matrix resultieren.

Zur Untersuchung der Bindungseigenschaften wurde prokaryonter DNA aus DNA-Gemisch von *Staphylococcus aureus* und humaner DNA unter Verwendung des direkt gekoppeltem CpGbP-181-Proteins an CNBr-Sepharose bzw. unter Verwendung des indirekt über einen Diaminohexyl-Spacer (AH) gekoppeltem CGBP181 Proteins an Sepharose (im folgenden AHSepharose) angereichert.

Zunächst wurde die AH-Sepharose nach Zugabe von Glutaraldehyd 15 Minuten bei Raumtemperatur inkubiert. Anschließend wurde die AH-Sepharose mit 0,1 molarem Na2HPO4gewaschen. Jetzt wurden 0,24 mg des CGBP181 -Proteins auf die Matrix gegeben. Die Bindung des CGBP181 -Proteins an die AH-Sepharose wurde durch eine Inkubation während 2 Stunden bei Raumtemperatur erreicht. Das überschüssige CGBP181 -Protein wurde entfernt.

Nach anschließendem Waschen der CGBP181 AH-Sepharose mit 0,1 molarem Na₂HPO₄ und Zugabe von 0,1 molarem Glycin, wurde die CGBP181 -AH-Sepharose zur Absättigung freier Bindungsstellen 2 Stunden bei Raumtemperatur inkubiert. Danach wurde wiederum die CGBP181 -AH-Sepharose mit 0,1 molarem Na₂HPO₄ gewaschen. Zur Reduktion der Schiffschen Base und Stabilisierung der Bindung wurde die CGBP181 AH-Sepharose mit Natriumborhydrid versetzt und 1 Stunde bei Raumtemperatur inkubiert.

Danach wurde die CGBP181 AH-Sepharose mit 0.1 molarem Na₂HPO₄ gewaschen.

Die Lagerfähigkeit der CGBP181 -AH-Sepharose bei 4°C wird durch Zugabe von 20% Ethanol erreicht Danach wurde die CGBP181 AH-Sepharose in Saulen portioniert. Die mit der CGBP181 AH-Sepharose präparierten Säulen wurden anschließend mit TRIS-Puffer gewaschen und standen für die Trennung/Anreicherung von nicht-methylierte CpG-Motive enthaltende DNA zur Verfügung.

### 2) Anreicherung des DNA-Gemisches, anschließende Elution der prokaryonten DNA und Konzentrationsbestimmung der prokaryonten DNA durch PCR

Als DNA-Gemisch bestand jeweils aus 330 ng humaner DNA bzw. 150 ng prokaryonter DNA (*Staphylococcus aureus* DNA). Die DNA-Gemische wurde auf die mit CNBr-Sepharose bzw. mit AH-Sepharose präparierten Säulen gegeben und 1 Minute bei Raumtemperatur inkubiert. Danach wurden die Säulen zentrifugiert und mit 100 µl TRIS-Puffer (10 µM. pH 7) gewaschen. Der Wasch- und Zentrifugationsschritt wurde 5 mal wiederholt

Der Überstand wurde vorsichtig abgenommen und anschließend jeweils 100 µl Elutionspuffer (10 µM TRIS-Puffer, 0,5 M NaCl, pH 7) auf die Säulen gegeben und zentrifugiert. Der Elutionsschritt wurde 5 mal wiederholt. Jetzt wurden die einzelnen Fraktionen jeder Probe durch Zugabe von 10 µl 3 M Natriumacetat und 250 µl Ethanol mit anschließendem Mischen und Zentrifugieren (15 min bei 15000g) gefällt. Der Überstand wurde vorsichtig abgegossen und das Pellet mit 1 µl Ethanol (70%) gewaschen und 5 Minuten bei 15000 g zentrifugiert. Anschleißend wurde wieder der Überstand entfernt, das Pellet in einer Vakuumzentrifuge getrocknet und in 30 µl DEPC-Wasser aufgenommen. Hiervon wurden jeweils 5 µl für den PCR-Nachweis verwendet.

Für die PCR wurden Universalprimer für das 16s RNA Gen verwendet. Nach Durchführung der PCR wurden jeweils 15 µl der einzelnen Fraktionen auf ein 2%-ges Agarosegel gegeben.

Figur 9 (direkte Bindung des CGBP181- Proteins an CNBr-Sepharose) und Figur 10 (indirekte Bindung des CGBP181 Proteins über eine Spacer (AH) an Sepahrose) zeigen die Ergebnisse der PCR für die einzelnen Fraktionen. Es zeigt sich deutlich, dass durch die Verwendung des AH-Spacer mehr prokayronte DNA angereichert werden konnte (Fraktion 1, Elutionsfraktion). Diese charakteristische Verbesserung der Bindungseigenschaften ist für die erfindungsgemäßen Verfahren ausnutzbar.

## Patentansprüche

1. Verfahren zur Abtrennung und/oder Anreicherung prokaryotischer DNA mit den Schritten:
a) Kontaktieren mindestens einer in Lösung befindlichen prokaryotischen DNA mit einem spezifisch prokaryotische DNA bindenden Protein, das eine Aminosäuresequenz gemäß SEQ ID NO: 2 aufweist, wodurch ein Protein-DNA-Komplex gebildet wird, wobei das Protein in der Lage ist, nicht-methylierte CpG-Motive zu erkennen, und
b) Separation des Komplexes.

2. Verfahren gemäß Anspruch 1, wobei sich an die Separation ein Schritt zur Abtrennung der DNA vom Protein aus dem Komplex anschließt.

3. Verfahren gemäß einem der vorgegangenen Ansprüche, wobei das Protein an einen Träger gebunden ist.

4. Verfahren gemäß Anspruch 3, wobei das Protein direkt an den Träger gebunden ist.

5. Verfahren gemäß Anspruch 3, wobei das Protein über einen gegen das Protein gerichteten Antikörper an den Träger gebunden ist.

6. Verfahren gemäß Anspruch 3, wobei das Protein über einen Spacer an einen Träger gebunden ist.

7. Verfahren gemäß Anspruch 6, wobei ein Diaminohexan-Rest als Spacer verwendet wird.

8. Verfahren gemäß einem der Ansprüche 3 bis 6, wobei der Träger als Matrix, Mikropartikel oder Membran ausgebildet ist.

9. Verfahren nach Anspruch 8, wobei als Matrix Sepharose verwendet wird.

10. Verfahren gemäß einem der vorhergehenden Ansprüche, wobei die Separation mittels eines gegen das Protein gerichteten Antikörper oder Antiserum erfolgt.

11. Verfahren gemäß einem der Ansprüche 1 bis 9, wobei die Separation mittels Elektrophorese erfolgt.

12. Verfahren gemäß einem der vorhergehenden Ansprüche, wobei die Lösung ein Gemisch aus eukaryotischer und prokaryotischer DNA enthält.

13. Verfahren- nach Anspruch 12, wobei es sich bei der prokaryotischen DNA um bakterielle DNA handelt.

14. Verfahren gemäß Anspruch 12 oder 13, wobei die Lösung eine Körperflüssigkeit oder davon abgeleitet ist, insbesondere Vollblut, Serum, Plasma, Zellpräparationen aus Vollblut, Urin, Liquor, Pleural-, Perikardial-, Peritoneal-, Synovialflüssigkeit und bronchoalveoläre Lavage.

15. Verfahren nach einem der Ansprüche 12 bis 14, wobei die Separation mittels eines Filters erzielt wird, welcher entsprechende DNA-Protein-Komplexe herausfiltert und wobei das Protein auf einer Filtermatrix immobilisiert ist.

16. Verfahren nach einem der Ansprüche 1 bis 15 zur Anwendung in der Umwelttechnik, der Wasser- und Abwasserwirtschaft sowie der Klimatechnik.

17. Verfahren nach einem der Ansprüche 1 bis 15, wobei weiterhin nach Schritt b) als Schritt c) die prokaryotische DNA amplifiziert wird.

18. Verfahren nach Anspruch 17, mit den Schritten:
a) Isolierung der prokaryotischen DNA aus dem Protein DNA-Komplex,
b) Denaturierung der doppelsträngigen DNA,
c) Hybridisierung der Einzelstränge der DNA mit komplementären Primern,
d) Generierung von Doppelstrangfragmenten über Reaktion mit Polymerasen und
e) Wiederholung dieser Schritte zum gewünschten Amplifikationsgrad.

19. Verfahren nach Anspruch 18, mit den Schritten:
a) Klonierung der isolierten prokaryotischen DNA-Sequenzen in Vektoren,
b) Transformation geeigneter Wirtszellen mit diesen Vektoren,
c) Kultivieren dieser transformierten Zellen,
d) Isolation der Vektoren aus diesen Zellen und
e) Isolierung der DNA.

20. Verwendung eines Kits, enthaltend mindestens ein spezifisch prokaryotische DNA bindendes Protein, das eine Aminosäuresequenz gemäß SEQ ID NO: 2 aufweist, zur Abtrennung und/oder Anreicherung prokaryotischer DNA mittels eines Verfahrens nach einem der Ansprüche 1 bis 19.

21. Verfahren zur Abtrennung und/oder Anreicherung von nicht-methylierter genomischer DNA aus einem Gemisch von nicht-methylierter genomischer und methylierte genomischer DNA mit den Schritten:
a) Kontaktieren einer in Lösung befindlichen nicht-methylierten genomischen DNA mit einem spezifisch nicht-methylierte DNA bindenden Protein, das eine Aminosäuresequenz gemäß SEQ ID NO: 2 aufweist, wodurch ein Protein-DNA-Komplex gebildet wird, und
b) Separation des Komplexes.

22. Verfahren nach Anspruch 21 zur Diagnose von Krankheiten, die ein spezifisches Methylierungsmuster der genomischen DNA aufweisen, insbesondere Krebserkrankungen.

## Claims

1. A method of separating and/or enriching prokaryotic DNA, including the steps of:
a) contacting at least one prokaryotic DNA, present in solution, with a protein which specifically binds prokaryotic DNA and includes an amino acid sequence acccording to SEQ ID No. 2, thereby forming a protein-DNA complex, wherein the protein is capable of recognizing non-methylated CpG motifs, and
b) separation of said complex.

2. The method according to claim 1, wherein separation is followed by a step for separating the DNA from the protein of the complex.

3. The method according to any one of the preceding claims, wherein the protein is bound to a carrier.

4. The method according to claim 3, wherein the protein is bound directly to the carrier.

5. The method according to claim 3, wherein the protein is bound to the carrier via an antibody directed against the protein.

6. The method according to claim 3, wherein the protein is bound to the carrier via a spacer.

7. The method according to claim 6, wherein a diamino hexane residue is used as the spacer.

8. The method according to any one of claims 3 to 6, wherein the carrier is provided as a matrix, microparticles, or a membrane.

9. The method according to claim 8, wherein sepharose is used as the matrix.

10. The method according to any one of the preceding claims, wherein separation is effected by means of an antibody or antiserum directed against the protein.

11. The method according to any one of claims 1 to 9, wherein separation is effected by means of electrophoresis.

12. The method according to any one of the preceding claims, wherein the solution contains a mixture of eukaryotic and prokaryotic DNA.

13. The method according to claim 12, wherein the prokaryotic DNA is bacterial DNA.

14. The method according to claim 12 or 13, wherein the solution is a body fluid or is derived therefrom, in particular whole blood, serum, plasma, cell preparations from whole blood, urine, liquor, pleural liquid, pericardial liquid, peritoneal liquid, synovial liquid or bronchoalveolar lavage.

15. The method according to any one of claims 12 to 14, wherein separation is achieved by means of a filter which filters out corresponding DNA-protein complexes and wherein the protein is immobilized to a filter matrix.

16. The method according to any one of claims 1 to 15 for use in environmental technology, water and sewage management, and climate technology.

17. The method according to any one of claims 1 to 15, wherein after step b) the prokaryotic DNA is furthermore amplified in a step c).

18. The method according to claim 17, including the steps of:
a) isolating the prokaryotic DNA from the protein-DNA complex,
b) denaturating the double-stranded DNA,
c) hybridizing the individual strands of the DNA with complementary primers,
d) generating double-strand fragments via reaction with polymerases, and
e) repeating these steps up to the desired degree of amplification.

19. The method according to claim 18, including the steps of:
a) cloning the isolated prokaryotic DNA sequences into vectors,
b) transforming suitable host cells with these vectors,
c) cultivating these transformed cells,
d) isolating the vectors from these cells, and
e) isolating the DNA.

20. Use of a kit containing at least one protein which specifically binds prokaryotic DNA and includes an amino acid sequence acccording to SEQ ID No. 2, for enriching and/or separating prokaryotic DNA by means of a method according to any one of claims 1 to 19.

21. A method of separating and/or enriching non-methylated genomic DNA from a mixture of non-methylated genomic and methylated genomic DNA, including the steps of:
a) contacting a non-methylated genomic DNA, present in solution, with a protein which specifically binds non-methylated DNA and includes an amino acid sequence acccording to SEQ ID No. 2, thereby forming a protein-DNA complex, and
b) separation of said complex.

22. The method according to claim 21 for diagnosing disorders presenting a specific methylation pattern of the genomic DNA, in particular cancerous disorders.

## Revendications

1. Procédé de séparation et/ou d'enrichissement d'ADN procaryote, comportant les étapes suivantes :
a) mise en contact d'au moins un ADN procaryote se trouvant en solution avec une protéine liant d'une manière spécifique l'ADN procaryote, protéine qui présente une séquence d'acides aminés selon SEQ ID N° 2, ce qui forme un complexe protéine-ADN, la protéine étant à même de reconnaître les motifs CpG non méthylés, et
b) séparation du complexe.

2. Procédé selon la revendication 1, dans lequel la séparation est suivie d'une étape consistant à séparer l'ADN de la protéine provenant du complexe.

3. Procédé selon l'une des revendications précédentes, dans lequel la protéine est liée à un support.

4. Procédé selon la revendication 3, dans lequel la protéine est directement liée au support.

5. Procédé selon la revendication 3, dans lequel la protéine est liée au support par l'intermédiaire d'un anticorps dirigé contre la protéine.

6. Procédé selon la revendication 3, dans lequel la protéine est liée à un support par l'intermédiaire d'un espaceur.

7. Procédé selon la revendication 6, dans lequel on utilise en tant qu'espaceur un résidu diaminohexane.

8. Procédé selon l'une des revendications 3 à 6, dans lequel le support est configuré sous forme d'une matrice, de microparticules ou d'une membrane.

9. Procédé selon la revendication 8, dans lequel on utilise du sépharose en tant que matrice.

10. Procédé selon l'une des revendications précédentes, dans lequel la séparation a lieu à l'aide d'un anticorps ou d'un antisérum dirigé contre la protéine.

11. Procédé selon l'une des revendications 1 à 9, dans lequel la séparation s'effectue par électrophorèse.

12. Procédé selon l'une des revendications précédentes, dans lequel la solution contient un mélange d'ADN eucaryote et d'ADN procaryote.

13. Procédé selon la revendication 12, dans lequel, pour ce qui concerne l'ADN procaryote, il s'agit d'un ADN bactérien.

14. Procédé selon la revendication 12 ou 13, dans lequel la solution est un fluide corporel ou en dérive, en particulier du sang total, du sérum, du plasma, des préparations cellulaires de sang total, de l'urine, du liquide céphalo-rachidien, du fluide pleural, péricardique, péritonéale, synovial et un lavage broncho-alvéolaire.

15. Procédé selon l'une des revendications 12 à 14, dans lequel la séparation est réalisée à l'aide d'un filtre qui extrait par filtration les complexes ADN-protéine correspondants, la protéine étant immobilisée sur une matrice filtrante.

16. Procédé selon l'une des revendications 1 à 15, pour utilisation dans les techniques de protection de l'environnement, la gestion de l'eau et des eaux résiduaires, ainsi qu'en climatisation.

17. Procédé selon l'une des revendications 1 à 15, dans lequel l'ADN procaryote est en outre amplifié après l'étape b), sous forme d'une étape c).

18. Procédé selon la revendication 17, comportant les étapes suivantes :
a) isolement de l'ADN procaryote d'avec le complexe protéine-ADN,
b) dénaturation de l'ADN double brin,
c) hybridation des simples brins de l'ADN avec des amorces complémentaires,
d) production de fragments double brin par réaction avec des polymérases, et
e) répétition de ces étapes jusqu'à atteindre le degré souhaité d'amplification.

19. Procédé selon la revendication 18, comportant les étapes suivantes :
a) clonage des séquences d'ADN procaryote isolées dans des vecteurs,
b) transformation de cellules hôtes appropriées à l'aide de ces vecteurs,
c) culture de ces cellules transformées,
d) isolement des vecteurs à partir de ces cellules, et
e) isolement de l'ADN.

20. Utilisation d'une trousse contenant au moins une protéine liant d'une manière spécifique l'ADN procaryote, protéine qui comprend une séquence d'acides aminés selon SEQ ID N° 2, pour la séparation et/ ou l'enrichissement de l'ADN procaryote à l'aide d'un procédé selon l'une des revendications 1 à 19.

21. Procédé pour séparer et/ ou enrichir un ADN génomique non méthylé, à partir d'un mélange d'ADN génomique non méthylé et d'ADN génomique méthylé, comportant les étapes suivantes :
a) mise en contact d'un ADN génomique non méthylé se trouvant en solution avec une protéine liant d'une manière spécifique l'ADN non méthylé, protéine qui présente une séquence d'acides aminés selon SEQ ID N° 2, ce qui va former un complexe protéine-ADN, et
b) séparation du complexe.

22. Procédé selon la revendication 21 pour le diagnostic de maladies qui présentent un modèle spécifique de méthylation de l'ADN génomique, en particulier les maladies cancéreuses.


### SEQUENZPROTOKOLL

<110> SIRS-Lab GmbH
<120> Verfahren zur Anreicherung/Trennung von prokaryonter DNA mittels eines Proteins, das nicht methylierte CpG-Motive enthaltende DNA spezifisch bindet
<130> Pat 3696/29-PCT
<140>
   <141>
<160> 8
<170> PatentIn Ver. 2.1
<210> 1
   <211> 543
   <212> DNA
   <213> Homo sapiens
<220>
   <221> CDS
   <222> (1)..(561)
<400> 1
   ggt gga ggg cgc aag agg cct gtc cct gat cca aac ctg cag cgc cgg     48
   Gly Gly Gly Arg Lys Arg Pro Val Pro Asp Pro Asn Leu Gln Arg Arg
     1               5                  10                  15
   gca ggg tca ggg aca ggg gtt ggg gcc atg ctt gct cgg ggc tct gct     96
   Ala Gly Ser Gly Thr Gly Val Gly Ala Met Leu Ala Arg Gly Ser Ala
                20                  25                  30
   tcg ccc cac aaa tcc tct ccg cag ccc ttg gtg gcc aca ccc agc cag    144
   Ser Pro His Lys Ser Ser Pro Gln Pro Leu Val Ala Thr Pro Ser Gln
            35                  40                  45
   cat cac cag cag cag cag cag cag atc aaa cgg tca gcc cgc atg tgt    192
   His His Gln Gln Gln Gln Gln Gln Ile Lys Arg Ser Ala Arg Met Cys
        50                  55                  60
   ggt gag tgt gag gca tgt cgg cgc act gag gac tgt ggt cac tgt gat    240
   Gly Glu Cys Glu Ala Cys Arg Arg Thr Glu Asp Cys Gly His Cys Asp
    65                  70                  75                  80
   ttc tgt cgg gac atg aag aag ttc ggg ggc ccc aac aag atc cgg cag    288
   Phe Cys Arg Asp Met Lys Lys Phe Gly Gly Pro Asn Lys Ile Arg Gln
                    85                  90                  95
   aag tgc cgg ctg cgc cag tgc cag ctg cgg gcc cgg gaa tcg tac aag    336
   Lys Cys Arg Leu Arg Gln Cys Gln Leu Arg Ala Arg Glu Ser Tyr Lys
               100                 105                 110
   tac ttc cct tcc tcg ctc tca cca gtg acg ccc tca gag tcc ctg cca    384
   Tyr Phe Pro Ser Ser Leu Ser Pro Val Thr Pro Ser Glu Ser Leu Pro
           115                 120                 125
   agg ccc cgc cgg cca ctg ccc acc caa cag cag cca cag cca tca cag    432
   Arg Pro Arg Arg Pro Leu Pro Thr Gln Gln Gln Pro Gln Pro Ser Gln
       130                 135                 140
   aag tta ggg cgc atc cgt gaa gat gag ggg gca gtg gcg tca tca aca    480
   Lys Leu Gly Arg Ile Arg Glu Asp Glu Gly Ala Val Ala Ser Ser Thr
   145                 150                 155                 160
   gtc aag gag cct cct gag gct aca gcc aca cct gag cca ctc tca gat    528
   Val Lys Glu Pro Pro Glu Ala Thr Ala Thr Pro Glu Pro Leu Ser Asp
                   165                 170                 175
   gag gac cta cct ctg                                                543
   Glu Asp Leu Pro Leu
               180
<210> 2
   <211> 181
   <212> PRT
   <213> Homo sapiens
<400> 2
   Gly Gly Gly Arg Lys Arg Pro Val Pro Asp Pro Asn Leu Gln Arg Arg
   1               5                   10                  15
   Ala Gly Ser Gly Thr Gly Val Gly Ala Met Leu Ala Arg Gly Ser Ala
               20                  25                  30
   Ser Pro His Lys Ser Ser Pro Gln Pro Leu Val Ala Thr Pro Ser Gln
           35                  40                  45
   His His Gln Gln Gln Gln Gln Gln Ile Lys Arg Ser Ala Arg Met Cys
       50                  55                  60
   Gly Glu Cys Glu Ala Cys Arg Arg Thr Glu Asp Cys Gly His Cys Asp
   65                  70                  75                  80
   Phe Cys Arg Asp Met Lys Lys Phe Gly Gly Pro Asn Lys Ile Arg Gln
                   85                  90                  95
   Lys Cys Arg Leu Arg Gln Cys Gln Leu Arg Ala Arg Glu Ser Tyr Lys
               100                 105                 110
   Tyr Phe Pro Ser Ser Leu Ser Pro Val Thr Pro Ser Glu Ser Leu Pro
           115                 120                 125
   Arg Pro Arg Arg Pro Leu Pro Thr Gln Gln Gln Pro Gln Pro Ser Gln
       130                 135                 140
   Lys Leu Gly Arg Ile Arg Glu Asp Glu Gly Ala Val Ala Ser Ser Thr
   145                 150                 155                 160
   Val Lys Glu Pro Pro Glu Ala Thr Ala Thr Pro Glu Pro Leu Ser Asp
                   165                 170                 175
   Glu Asp Leu Pro Leu
<210> 3
   <211> 28
   <212> DNA
   <213> Künstliche Sequenz
<220>
   <223> Beschreibung der künstlichen Sequenz: Primer
<400> 3
   ggatccggtg gagggcgcaa gaggcctg 28
<210> 4
   <211> 27
   <212> DNA
   <213> Künstliche Sequenz
<220>
   <223> Beschreibung der künstlichen Sequenz: Primer
<400> 4
   aagcttagag gtaggtcctc atctgag 27
<210> 5
   <211> 26
   <212> DNA
   <213> Künstliche Sequenz
<220>
   <223> Beschreibung der künstlichen Sequenz: Primer
<400> 5
   agcatacaag caaatttttt acaccg 26
<210> 6
   <211> 24
   <212> DNA
   <213> Künstliche Sequenz
<220>
   <223> Beschreibung der künstlichen Sequenz: Primer
<400> 6
   gttctgttat tgacacccgc aatt 24
<210> 7
   <211> 24
   <212> DNA
   <213> Künstliche Sequenz
<220>
   <223> Beschreibung der künstlichen Sequenz: Primer
<400> 7
   ccttcctaat aatcctgcgg atgt 24
<210> 8
   <211> 28
   <212> DNA
   <213> Künstliche Sequenz
<220>
   <223> Beschreibung der künstlichen Sequenz: Primer
<400> 8
   ctgaaggtag cattagtctt tgataacg 28